# EUROPÄISCHE PATENTSCHRIFT

(11) **EP 4 669 965 B1**
(45) Veröffentlichungstag und Bekanntmachung des Hinweises auf die Patenterteilung: **26.08.2026**
(21) Anmeldenummer: 23706335.9
(22) Anmeldetag: 20.02.2023
(51) Int. Cl.: G01N 33/543, G01N 33/58, C12Q 1/6816, B03C 1/28, B03C 1/01

(54) **VERFAHREN ZUM DIREKTEN NACHWEIS DES VORHANDENSEINS EINER PRÜFSUBSTANZ**
METHOD FOR DIRECT DETECTION OF THE PRESENCE OF A TEST SUBSTANCE
MÉTHODE DE DÉTECTION DIRECTE DE LA PRÉSENCE D'UNE SUBSTANCE D'ESSAI

(43) Veröffentlichungstag der Anmeldung: 31.12.2025
(73) Patentinhaber: Odefey, Constantin, 22085 Hamburg (DE); STILLER, Olaf, 47802 Krefled (DE)
(72) Erfinder: ODEFEY, Constantin, 22085 Hamburg (DE)
(74) Vertreter: Hauck Patent- und Rechtsanwälte PartmbB
(86) Internationale Anmeldenummer: PCT/EP2023/054157
(87) Internationale Veröffentlichungsnummer: WO 2024/175169

(56) Entgegenhaltungen:
- WO-A1-2004/009848
- WO-A1-2018/140719
- WO-A2-2007/095279
- US-A1- 2015 316 545
- WU CONNIE ET AL: "High-Throughput, High-Multiplex Digital Protein Detection with Attomolar Sensitivity", ACS NANO, vol. 16, no. 1, 14 January 2022 (2022-01-14), US, pages 1025 - 1035, XP093070554, ISSN: 1936-0851, DOI: 10.1021/acsnano.1c08675
- TEKIN H. CUMHUR ET AL: "Attomolar protein detection using a magnetic bead surface coverage assay", LAB ON A CHIP, vol. 13, no. 6, 1 January 2013 (2013-01-01), UK, pages 1053, XP093076199, ISSN: 1473-0197, DOI: 10.1039/c3lc41285g

## Beschreibung

Die vorliegende Erfindung betrifft ein Verfahren zum direkten Nachweis des Vorhandenseins einer Prüfsubstanz in einem Prüffluid.

Verfahren zum Nachweis des Vorhandenseins von Prüfsubstanzen in einem Prüffluid werden in Chemie, (Mikro-)biologie, medizinischer Analytik und Bio-Chemie vielfältig eingesetzt. Eine typische Prüfsubstanz ist ein krankheitserregendes Virus oder Bakterium, das von einem entsprechenden Abstrich eines Patienten stammt und in Flüssigkeit eingemischt wurde. Auch der Nachweis von giftigen Stoffen in einem Prüffluid ist ein typisches Anwendungsgebiet solcher Verfahren. Bei Fluiden kann es sich insbesondere um homogene (einphasige) Flüssigkeiten handeln, bestehend aus einer einzigen flüssigen Komponente oder aus einer homogenen Mischung mehrerer flüssiger Komponenten (Lösungen). Ferner können Fluide heterogene (mehrphasige) Gemische einer Flüssigkeit mit einer weiteren Flüssigkeit (Emulsionen) oder einem Feststoff (Suspensionen) sein.

Zum Nachweis des Vorhandenseins einer Prüfsubstanz werden üblicherweise Marker-Teilchen, wie z.B. Farbstoffe, verwendet, die eine physikalische Eigenschaft des Prüffluids in Abhängigkeit des Vorhandenseins der Prüfsubstanz verändern. Dazu wird das Marker-Teilchen dem Prüffluid zugesetzt. In der Folge sind die physikalischen Eigenschaften des Prüffluids, z.B. ihre Farbe oder elektrische Leitfähigkeit, verändert. Das Marker-Teilchen ist so ausgebildet, dass es direkt oder indirekt an ein Prüfsubstanz-Teilchen bindet. Nach der Zugabe des Marker-Teilchens wird durch geeignete Vorkehrungen das Prüfsubstanz-Teilchen und das daran gebundene Marker-Teilchen aus dem Prüffluid entfernt. Im Falle, dass allerdings keine Prüfsubstanz in dem Prüffluid vorhanden war, verbleibt das Marker-Teilchen in dem Prüffluid. Die physikalischen Eigenschaften des Prüffluids sind dann durch das Marker-Teilchen entsprechend verändert und diese Veränderung ist einfach zu erfassen, bspw. anhand einer Analyse der Farbe des Prüffluids.

Um das Prüfsubstanz-Teilchen aus dem Prüffluid zu entfernen, können bspw. Nanobeads eingesetzt werden. Nanobeads sind kleine Teilchen verschiedenster Formen, welche über eine funktionalisierte Oberfläche verfügen. Die funktionalisierte Oberfläche kann in Abhängigkeit der zu detektierenden Prüfsubstanzen mit passenden Bindungsstellen versehen werden. Die Bindungsstellen bilden beim Zusammenkommen eines Nanobeads und dem Prüfsubstanz-Teilchen mit den komplementären Bindungsstellen des Prüfsubstanz-Teilchens eine Bindung aus. Dies kann bspw. eine Antigen-Antikörper-Bindung sein. Ferner sind Nanobeads typischerweise eisenhaltig und somit magnetisch. Durch das Aufbringen einer magnetischen Kraft können Nanobeads und daran gebundene Prüfsubstanz-Teilchen aus dem Prüffluid entfernt werden.

Nachteilig ist, dass beim Testen eines Prüffluids auf Vorhandensein des Prüfsubstanz-Teilchens das Marker-Teilchen nicht in dem Prüffluid verbleibt, wenn das Prüfsubstanz-Teilchen vorhanden ist. Nur wenn kein Prüfsubstanz-Teilchen in dem Prüffluid vorhanden ist, verbleibt das Marker-Teilchen in dem Prüffluid und der Test zeigt ein positives Ergebnis. Es handelt sich folglich um einen reziproken Test, bei dem ein positives Testergebnis ein Nichtvorhandensein des Prüfsubstanz-Teilchens kennzeichnet.

Aus EP 3 147 028 A1 ist ein Verfahren zur magnetischen Trennung von Nanobeads aus einem ersten Fluid bekannt geworden, das die folgenden Schritte umfasst: Das Bereitstellen des ersten Fluids mit den darin befindlichen Nanobeads; Einführen eines Hülsenkörpers in das erste Fluid, wobei in dem Hülsenkörper ein erster Magnet angeordnet ist, der entlang einer Längsachse des Hülsenkörpers verschiebbar ist; Sammeln von Nanobeads an der Hülsenwand durch die Kraftwirkung eines ersten magnetischen Feldes des ersten Magneten; Entfernen des Hülsenkörpers mit den gesammelten Nanobeads aus dem ersten Fluid; Einführen des Hülsenkörpers mit den gesammelten Nanobeads in ein zweites Fluid; und Bereitstellen eines magnetischen Feldes durch eine ein zweites magnetisches Feld bereitstellende Vorrichtung, dessen magnetische Polausrichtung der Polausrichtung des ersten Magneten in dem Hülsenkörper entgegengesetzt ist, so dass der erste Magnet in dem Hülsenkörper von dem magnetischen Feld abgestoßen und in dem Hülsenkörper verschoben wird, wobei die ein zweites magnetisches Feld bereitstellende Vorrichtung und der Hülsenköper in einer Richtung senkrecht zur Längsachse nicht überlappen.

US2015/316545 offenbart einen Immunassay mit zwei magnetischen Trennungen unter Verwendung von zwei verschiedenen magnetischen Nanobeads und biotinylierten Goldnanopartikeln.

Ausgehend hiervon ist die Aufgabe der Erfindung das Bereitstellen eines nanobeadbasierten Test-Verfahrens, das beim Vorhandensein einer Prüfsubstanz in einem Prüffluid positiv anspricht.

Gelöst wird diese Aufgabe durch ein Verfahren gemäß Anspruch 1. Vorteilhafte Ausführungsarten sind in den Unteransprüchen und in der nachfolgenden Beschreibung angegeben.

Das erfindungsgemäße Verfahren zum Nachweis einer Prüfsubstanz in einem Prüffluid umfasst die folgenden Schritte:
i. Bereitstellen eines ersten Fluids, dem Prüffluid, in dem in einem ersten Fall ein Prüfsubstanz-Teilchen vorhanden ist und in einem zweiten Fall das Prüfsubstanz-Teilchen nicht vorhanden ist,
   o wobei das Prüfsubstanz-Teilchen Bindungsstellen erster Art aufweist,
ii. Bereitstellen von
   o einem ersten Nanobead-Fluid, das ein erstes magnetisches Nanobead mit einer komplementären Bindungsstelle erster Art enthält,
   o einem zweiten Nanobead-Fluid, das ein zweites magnetisches Nanobead mit einer Bindungsstelle zweiter Art enthält,
   o einem dritten Nanobead-Fluid, das ein nicht-magnetisches Nanobead mit einer komplementären Bindungsstelle erster Art, einer komplementären Bindungsstelle zweiter Art und einer komplementären Bindungsstelle dritter Art enthält,
   o einem Marker-Fluid, das ein Marker-Teilchen mit einer Bindungsstelle dritter Art enthält,
   o wobei bei Vorhandensein entsprechender Bindungspartner in demselben Fluid
      ▪ eine Bindungsstelle erster Art eines Prüfsubstanz-Teilchens mit einer komplementären Bindungsstelle erster Art eines magnetischen Nanobeads erster Art und eines nicht-magnetischen Nanobeads eine Bindung erster Art ausbildet,
      ▪ eine Bindungsstelle zweiter Art eines magnetischen Nanobeads zweiter Art mit einer komplementären Bindungsstelle zweiter Art eines nicht-magnetischen Nanobeads eine Bindung zweiter Art ausbildet,
      ▪ eine Bindungsstellen dritter Art eines Marker-Teilchens mit einer komplementären Bindungsstelle dritter Art eines nicht-magnetischen Nanobeads eine Bindung dritter Art ausbildet,
   iii. Erstellen eines zweiten Fluids,
      o indem das Prüffluid mit dem ersten Nanobead-Fluid vermischt wird und zumindest das erste magnetische Nanobead des resultierenden Fluids mit dem dritten Nanobead-Fluid vermischt wird, wonach das resultierende Fluid als zweites Fluid bezeichnet ist,
   iv. Entfernen des ersten magnetischen Nanobeads aus dem zweiten Fluid mittels einer ersten magnetischen Kraft,
      o wobei im ersten Fall das an das erste magnetische Nanobead gebundene Prüfsubstanz-Teilchen und das an das Prüfsubstanz-Teilchen gebundene, nicht-magnetische Nanobead ebenfalls aus dem zweiten Fluid entfernt werden
      o oder im zweiten Fall das nicht-magnetische Nanobead in dem zweiten Fluid verbleibt,
   v. Erstellen eines dritten Fluids, indem
      o das zweite Nanobead-Fluid und das Marker-Fluid in das zweite Fluid eingemischt werden, wonach das Gemisch als drittes Fluid bezeichnet ist,
   vi. Entfernen des zweiten magnetischen Nanobeads aus dem dritten Fluid mittels einer zweiten magnetischen Kraft,
   o wobei im ersten Fall aufgrund des Nichtvorhandenseins des nicht-magnetischen Nanobeads in dam dritten Fluid das Marker-Teilchen nicht indirekt an das zweite magnetische Nanobead gebunden ist, sodass es nach dem Entfernen des zweiten magnetischen Nanobeads in dem dritten Fluid verbleibt, oder
   o wobei im zweiten Fall das nicht-magnetische Nanobead an das zweite magnetische Nanobead gebunden ist und das Marker-Teilchen an das nicht-magnetische Nanobead gebunden ist, sodass das Marker-Teilchen zusammen mit dem zweiten magnetischen Nanobead und dem nicht-magnetischen Marker-Teilchen aus dem dritten Fluid entfernt wird.

Das erfindungsgemäße Verfahren ist durch die Verwendung des nicht-magnetischen Nanobeads und zwei verschiedenen magnetischen Nanobeads gekennzeichnet. Das nicht-magnetische Nanobead ermöglicht, dass bei Anwendung des Verfahrens die Anwesenheit des Marker-Teilchens in dem finalen, dritten Fluid direkt darauf hinweist, dass zu Beginn das Prüfsubstanz-Teilchen in dem Prüffluid vorhanden war. Ein auf dem erfindungsgemäßen Verfahren basierender Test zum Nachweis des Vorhandenseins der Prüfsubstanz in dem Prüffluid schlägt auf diese Weise positiv an, wenn das Prüfsubstanz-Teilchen in dem Prüffluid vorhanden war.

Das erste magnetische Nanobead dient dazu, bei Vorhandensein des Prüfsubstanz-Teilchens das nicht-magnetische Nanobead aus dem zweiten Fluid zu entfernen. Dies geschieht nach der Erstellung des zweiten Fluids, welches durch das Vermischen zumindest von Prüffluid und erstem Nanobead-Fluid sowie dem Vermischen des ersten magnetischen Nanobeads des resultierenden Gemischs mit dem dritten Nanobead-Fluid entsteht. Dabei bindet beim Vermischen von erstem Nanobead-Fluid und Prüffluid das erste magnetische Nanobead an das Prüfsubstanz-Teilchen, sofern dieses in dem Prüffluid enthalten ist, und beim Einbringen des ersten magnetischen Nanobeads in das dritte Nanobead-Fluid bindet das gegebenenfalls an das erste magnetisch gebundene Nanobead gebundene Prüfsubstanz-Teilchen an das nicht-magnetische Nanobead. Anschließend wird aus dem zweiten Fluid durch die erste magnetische Kraft das erste magnetische Nanobead entfernt. Sollte das Prüfsubstanz-Teilchen nicht vorhanden sein, verbleibt nach der Entfernung des ersten magnetischen Nanobeads aus dem zweiten Fluid das nicht-magnetische Nanobead in dem zweiten Fluid, weil es nicht direkt, sondern nur indirekt über das Prüfsubstanz-Teilchen an das erste magnetische Nanobead binden kann.

In der vorliegenden Anmeldung wird unter einem "Vermischen" "Einmischen" und "Zumischen" (nachfolgend zusammenfassend "Vermischen") von Fluiden das Zusammenbringen von Fluiden verstanden, sodass diese unmittelbar miteinander in Kontakt treten. Das Vermischen von Fluiden kann insbesondere durch Zusammengießen oder Übereinandergießen von Fluiden oder durch Einleiten eines Fluides in ein anderes Fluid erfolgen. Das Einleiten eines Fluides kann beispielsweise dadurch erfolgen, dass in ein bereitstehendes Fluid in einem Gefäß ein Fluid mittels einer Pipette hineingegeben wird. Vorzugsweise erfolgt das Vermischen von Fluiden derart, dass diese gleichmäßig ineinander verteilt werden, beispielsweise indem diese gerührt oder geschüttelt werden. Das Vermischen umfasst aber auch ein Zusammenbringen von Fluiden, bei denen diese ungleichmäßig über das insgesamt von den Fluiden eingenommene Volumen verteilt sind. Maßgeblich ist, dass die Fluide miteinander in Kontakt kommen, sodass in den verschiedenen Fluiden enthaltene Teilchen (z.B. Prüfsubstanz-Teilchen, magnetische Nanobeads, nicht-magnetische Nanobeads oder Marker-Teilchen) in Wechselwirkung miteinander treten und miteinander Bindungen eingehen können, sofern es sich um Bindungspartner im Sinne der Erfindung handelt.

Das zweite magnetische Nanobead dient dazu, bei ursprünglichem Nichtvorhandensein des Prüfsubstanz-Teilchens das Marker-Teilchen aus dem dritten Fluid zu entfernen. Dies geschieht nachdem durch das Einmischen des Marker-Teilchens und des zweiten magnetischen Nanobeads in das zweite Fluid aus dem zweiten Fluid das dritte Fluid wird. Ähnlich wie zuvor bei dem zweiten Fluid wird die zweite magnetische Kraft verwendet, um das zweite magnetische Nanobead aus dem dritten Fluid zu entfernen. Das Marker-Teilchen kann nicht direkt an das zweite magnetische Nanobead binden, sondern nur indirekt über das nicht-magnetische Nanobead, das sowohl an das zweite magnetische Nanobead als auch an das Marker-Teilchen binden kann. Das nicht-magnetische Nanobead ist allerdings nur in dem dritten Fluid vorhanden, wenn es nicht zuvor aus dem zweiten Fluid entfernt wurde, was nur der Fall ist, wenn es indirekt über das Prüfsubstanz-Teilchen an das erste magnetische Nanobead binden und mit diesem aus dem zweiten Fluid entfernt werden konnte. Das Marker-Teilchen wird also nur aus dem dritten Fluid entfernt, wenn das nicht-magnetische Nanobead in dem dritten Fluid vorhanden war, was wiederum nur der Fall ist, wenn das Prüfsubstanz-Teilchen ursprünglich nicht in dem Prüfsubstanz-Fluid vorhanden war.

Die Anwesenheit des Prüfsubstanz-Teilchens entscheidet folglich darüber, ob das nicht-magnetische Nanobead in dem dritten Fluid abwesend ist. Nur bei Abwesenheit des nicht-magnetischen Nanobeads in dem dritten Fluid, welche einer Anwesenheit des Prüfsubstanz-Teilchens in dem zweiten Fluid entspricht, verbleibt das Marker-Teilchen in dem dritten Fluid nach der Entfernung des zweiten magnetischen Nanobeads. Der Test wird daher auch als reziprok-reversibel bezeichnet.

Der Vorteil des erfindungsgemäßen Verfahrens ist, dass ein Verbleiben des Markers in dem dritten Fluid, d.h. ein positives Test-Ergebnis, direkt das ursprüngliche Vorhandensein des Prüfsubstanz-Teilchens in dem Prüffluid anzeigt. Hieraus ergeben sich Vorteile bei der Handhabung des Verfahrens beim Einsatz in einem Labor.

Ein auf dem Verfahren basierender Test schlägt nicht an, wenn eines der eingesetzten Teilchen einen Defekt aufweist, der dafür sorgt, dass der Test unbrauchbar ist. Beim Anschlagen des Tests kann sich ein Labor folglich darauf verlassen, dass ein Prüfsubstanz-Teilchen auch tatsächlich vorhanden ist und nicht nur ein falschpositives Anschlagen des Tests vorliegt.

Ferner eignet sich ein auf dem Verfahren beruhender Test besonders für Pooling-Untersuchungen. Bei solchen Untersuchungen wird überprüft, ob sich mehrere verschiedene potentielle Prüfsubstanzen (z.B. Bakterien oder Viren) in dem Prüffluid gleichzeitig befinden. Mit dem erfindungsgemäßen Verfahren ist es möglich, das Vorhandensein einer gesuchten, speziellen Prüfsubstanz direkt anzuzeigen, anstatt nur indirekt auszuschließen, dass keine der potentiellen Prüfsubstanzen in dem Prüffluid vorhanden war.

Darüber hinaus ist vorteilhaft, dass die nicht-magnetischen Nanobeads dazu verwendet werden, die Marker-Teilchen zu binden. Die nicht-magnetischen Nanobeads können so ausgebildet sein, dass sie mit sehr vielen Marker-Teilchen gleichzeitig eine Bindung eingehen. Auf diese Weise kann ein auf dem Verfahren basierender Test hochsensibel sein. Es reichen wenige nicht-magnetische Nanobeads aus, um eine große Menge Marker-Teilchen zu entfernen und damit ein einfach zu detektierendes Test-Signal zu erzeugen. Wenn bereits ein einzelnes nicht-magnetisches Nanobead infolge der Anwesenheit eines einzelnen Prüfsubstanz-Teilchens aus dem zweiten Fluid entfernt wird, verbleiben signifikant mehr Marker-Teilchen in dem Prüffluid als in einem Fall, in dem kein Prüfsubstanz-Teilchen anwesend ist und alle nicht-magnetischen Nanobeads in dem dritten Fluid zur Bindung von Marker-Teilchen verbleiben.

Gemäß einer Ausführungsart des Verfahrenswird zum Erstellen des zweiten Fluids das dritte Nanobead-Fluid mit dem Prüffluid und mit dem ersten Nanobead-Fluid vermischt.

Bei der Durchführung des Verfahrens gibt es Alternativen zum Erstellen der zweiten Fluids. In einer Alternative werden das erste Nanobead-Fluid, das die ersten magnetischen Nanobeads enthält, und das Prüffluid direkt mit dem dritten Nanobead-Fluid, das die nicht-magnetischen Nanobeads enhält, vermischt. Eine solche Durchführung des Verfahrens ist besonders einfach.

Gemäß einer Ausführungsart des Verfahrens wird zum Erstellen des zweiten Fluids
- als erstes das erste Nanobead-Fluid mit dem Prüffluid vermischt,
- als zweites das erste magnetische Nanobead durch eine dritte magnetische Kraft aus dem Prüffluid entfernt und
- als drittes das aus dem Prüffluid entfernte erste magnetische Nanobead durch eine vierte magnetische Kraft in das dritte Nanobead-Fluid eingebracht.

Neben der zuvor beschriebenen Alternative zur Erstellung des zweiten Fluids ist aber auch möglich, zuerst nur das erste Nanobead-Fluid mit dem Prüffluid zu vermischen und dann das erste magnetische Nanobead mit der ersten Magnetkraft wieder aus diesem Gemisch zu entfernen. Sollte ein Prüfsubstanz-Teilchen vorhanden sein, wird es dann gemeinsam mit dem ersten magnetischen Nanobead aus dem ursprünglichen Prüffluid entnommen. Das erste Nanobead und das gegebenenfalls daran gebundene Prüfsubstanz-Teilchen wird anschließend in das dritte Nanobead-Fluid eingeführt und kommt erst dann mit dem nicht-magnetischen Nanobead in Kontakt. Vorteilhaft ist hieran, dass keine Gefahr besteht, dass das nicht-magnetische Nanobead mit Verunreinigungen in dem Prüffluid reagiert. Außerdem kann das Gefäß des dritten Nanobead-Fluids klein sein, sodass die Konzentration der Prüfsubstanzen-Teilchen in diesem Fluid größer als in dem ursprünglichen Prüffluid ist. Auf diese Weise ist die Sensitivität eines auf dem Verfahren in dieser Alternative beruhenden Tests gesteigert.

Gemäß einer Ausführungsart des Verfahrens
- weist das Prüfsubstanzteilchen zwei oder mehr Bindungsstellen erster Art auf,
- weist das erste magnetische Nanobead eine einzelne oder mehr komplementäre Bindungsstellen erster Art auf,
- weist das zweite magnetische Nanobead eine einzelne oder mehr Bindungsstellen zweiter Art auf,
- weist das nicht-magnetische Nanobead eine einzelne oder mehr komplementäre Bindungsstellen erster Art, eine einzelne oder mehr komplementäre Bindungsstellen zweiter Art, eine einzelne oder mehr komplementäre Bindungsstellen dritter Art auf sowie
- weist das Marker-Teilchen eine einzelne oder mehre Bindungsstellen dritter Art auf.

Üblicherweise weisen die beteiligten Teilchen mehrere Bindungsstellen bzw. komplementäre Bindungsstellen auf. Sofern die Teilchen nur eine Bindungsstelle aufweisen, ist die Herstellung aufwändig. Besonders das Aufweisen mehrerer komplementärer Bindungsstellen dritter Art bei lediglich einer komplementären Bindungsstelle erster Art am nicht-magnetischen Nanobead sorgt für ein günstiges Verhältnis von der Anzahl der Prüfsubstanz-Teilchen zu einer Veränderung der Konzentration der Marker-Teilchen in dem endgültigen Fluid. Dieses günstige Verhältnis ermöglicht, dass ein auf dem Verfahren basierender Test sehr sensitiv ist.

Gemäß einer Ausführungsart des Verfahrens
- enthält das Prüffluid ein einzelnes oder mehrere Prüfsubstanz-Teilchen, und/oder
- enthält das erste Nanobead-Fluid ein einzelnes oder mehrere erste magnetische Nanobeads und/oder
- enthält das zweite Nanobead-Fluid ein einzelnes oder mehrere zweites magnetische Nanobeads und/oder
- enthält das dritte Nanobead-Fluid ein einzelnes oder mehrere nicht-magnetische Nanobeads und/oder
- enthält das erste Marker-Fluid ein einzelnes oder mehrere Marker-Teilchen werden.

Üblicherweise werden von allen verwendeten Teilchen mehrere verwendet. So wird die Wahrscheinlichkeit gesteigert, dass es zu einer Ausbildung einer Bindung zwischen den jeweiligen Teilchen kommt. Es ist aber auch möglich, sofern lediglich ein Prüfsubstanz-Teilchen vorhanden ist, auch nur jeweils ein anderes der weiteren Teilchen zu verwenden, um dieses einzelne Prüfsubstanz-Teilchen nachzuweisen.

In einer weiteren Ausführungsart wird das Verfahren zum Nachweis der Prüfsubstanz in dem Prüffluid eingesetzt, wenn die Anzahl der Prüfsubstanz-Teilchen im femto- und/oder atto- und/oder zeptomoleren Bereich liegt. In einer weiteren Ausführungsart wird das Verfahren für Single-Molecule-Counting eingesetzt. Eine Anwendung bei einer größeren Anzahl an Prüfsubstanz-Teilchen ist auch möglich.

Gemäß einer Ausführungsart des Verfahrens ist es so, dass die Bindung erster Art eine Antikörper-Antigen-Bindung oder eine Oligonucleotid-Bindung oder eine Lipid-Bindung oder eine Glucose-Bindung ist.

Die Nanobeads können mit verschiedenen Bindungsstellen bzw. komplementären Bindungsstellen hergestellt werde. Hierdurch können viele verschiedene Prüfsubstanzen, die verschiedene Typen von Bindungsstellen erster Art aufweisen, mit dem erfindungsgemäßen Verfahren untersucht werden. Bei vielen Prüfsubstanzen, bspw. Viren oder Bakterien, wird zwischen Prüfsubstanz-Teilchen und erstem magnetischen Nanobead bzw. nicht-magnetischem Nanobead eine Bindung erster Art als Antikörper-Antigen-Bindung ausgebildet, aber bei anderen Prüfsubstanzen, bspw. giftigen Stoffen, kann es sich auch um eine Oligonucleotid-Bindung, Lipid-Bindung oder eine Glucose-Bindung handeln.

Gemäß einer Ausführungsart des Verfahrens ist es so, dass die Bindung zweiter Art eine Antikörper-Antigen-Bindung oder eine Oligonucleotid-Bindung oder eine Lipid-Bindung oder eine Glucose-Bindung ist.

Auch bei der Bindung zweiter Art sind verschiedene Ausprägungen umsetzbar. Wie das zweite magnetische Nanobead und das nicht-magnetische Nanobead bei der Herstellung bestückt werden, richtet sich danach, welchen äußeren Bedingungen das Verfahren ausgesetzt werden soll. Es wird durch die Möglichkeit, verschiedene Typen von Bindungen zweiter Art einsetzen zu können, somit flexibler.

Gemäß einer Ausführungsart des Verfahrens ist es so, dass die Bindung dritter Art eine Antikörper-Antigen-Bindung oder eine Oligonucleotid-Bindung oder eine Lipid-Bindung oder eine Glucose-Bindung ist.

Durch die Verwendung verschiedener Bindungen dritter Art kann eine große Anzahl möglicher Marker-Teilchen für das Verfahren genutzt werden, die über verschiedene Typen von Bindungsstellen dritter Art verfügen. So wird das Verfahren auf spezifische Gegebenheiten in einem Labor hinsichtlich der vorhandenen Analytik zum Nachweis des Marker-Teilchens abgestimmt.

Gemäß einer Ausführungsart des Verfahrens ist es so, dass das Marker-Teilchen ein farbiges Teilchen, ein leuchtendes Teilchen, ein fluoreszierendes Teilchen, ein die elektrische Leitfähigkeit einer Flüssigkeit beeinflussendes Teilchen oder ein radioaktives Teilchen ist.

In Abhängigkeit der zur Verfügung stehenden Analytik sind verschiedene Marker-Teilchen einsetzbar. Gebräuchlich sind Farbstoffe, welche das dritte Fluid einfärben und mit bloßem Auge erkennbar sind. Diese Farbstoffe können zur besseren Erkennbarkeit leuchten oder fluoreszieren, was bedeutet, dass sie bei einer Bestrahlung mit einer Lichtquelle leuchten. Vor allem fluoreszierende Farbstoffe können auch bei einer sehr geringen Konzentration bereits detektiert werden. Auch eine Änderung der Konzentration ist einfach erfassbar. Ferner ist auch eine Veränderung der elektrischen Leitfähigkeit einem Fluid in Abhängigkeit der Konzentration der entsprechenden, in dem Fluid enthaltenen Marker-Teilchen genau zu detektieren. Hierzu ausgeprägte Marker-Teilchen eigenen sich folglich auch sehr gut für das Verfahren. Ebenso sind radioaktive Marker-Teilchen genau nachweisbar und eignen sich somit als Marker-Teilchen.

Gemäß einer Ausführungsart des Verfahrens ist es so, dass
- sich in dem Prüffluid ein zusätzliches Prüfsubstanz-Teilchen mit einer alternativen Bindungsstelle erster Art befindet,
- eine zusätzliches erstes Nanobead-Fluid bereitgestellt wird, das ein alternatives erstes Nanobead mit einer alternativen komplementären Bindungsstelle erster Art enthält, und
- eine zusätzliches drittes Nanobead-Fluid bereitgestellt wird, das ein alternatives nicht-magnetisches Nanobead mit einer alternativen komplementären Bindungsstelle erster Art und einer alternativen komplementären Bindungsstelle dritter Art enthält, und
- ein zusätzliches Marker-Fluid bereitgestellt wird, das ein alternatives Marker-Teilchen mit einer alternativen Bindungsstelle dritter Art enthält,
- wobei das zusätzliche erste Nanobead-Fluid, das zusätzliche dritte Nanobead-Fluid und das zusätzliche Marker-Fluid in dem Verfahren bei denselben Schritten und in derselben Weise wie das erste Nanobead-Fluid, das dritte Nanobead-Fluid bzw. das Marker-Fluid eingesetzt werden.

Ein Pooling-Test, bei dem gleichzeitig das Vorhandensein von verschiedenen Sorten von Prüfsubstanzen untersucht wird, lässt sich vorteilhaft mit auf die jeweiligen Prüfsubstanzen abgestimmten Nanobeads durchführen. Dazu werden alternative Nanobeads mit alternativen Bindungsstellen eingesetzt, welche zum Nachweis einer zusätzlichen und zu der ursprünglichen Prüfsubstanz alternativen Prüfsubstanz geeignet sind. Die Durchführung des Verfahrens läuft bei Verwendung alternativer Nanobeads gleich wie zuvor beschrieben ab, nur dass mehrere Nanobead-Fluide gleichzeitig bei den jeweiligen Verfahrensschritten zum Einsatz kommen. Insbesondere kommt auch ein alternatives Marker-Fluid zum Einsatz, die ein Marker-Teilchen enthält, das nur an die alternativen, nicht-magnetischen Nanobeads bindet. Dieses nicht-magnetische Nanobead wiederum ist nur dazu in der Lage, mit einem Prüfsubstanz-Teilchen einer bestimmten Sorte eine Bindung auszubilden. Dadurch wird das Vorhandensein einer Prüfsubstanz einer gewissen Sorte auch durch ein entsprechendes Marker-Teilchen angezeigt. In Abhängigkeit des Vorhandenseins der jeweiligen, verschiedenen Prüfsubstanz-Sorten werden nur die jeweilig entsprechenden Marker-Teilchen in dem finalen Fluid verbleiben, sodass sich beispielsweise eine charakteristische Färbung des dritten Fluids ergibt, die darauf rückschließen lässt, welche Sorte von Prüfsubstanzen ursprünglich vorhanden und welche nicht vorhanden war.

Gemäß einer Ausführungsart des Verfahrens ist es so, dass
- das Prüffluid ein einzelnes oder mehrere zusätzliche Prüfsubstanz-Teilchen enthält, und/oder
- das erste zusätzliche Nanobead-Fluid ein einzelnes oder mehrere alternative erste Nanobeads enthält und/oder
- das dritte zusätzliche Nanobead-Fluid ein einzelnes oder mehrere alternative dritte Nanobeads enthält und/oder
- das zusätzliche Marker-Fluid ein einzelnes oder mehrere alternative Marker-Teilchen enthält.

Wie bei den nicht-alternativen Prüfsubstanz-Teilchen, Nanobeads und Marker-Teilchen können auch von den alternativen Prüfsubstanz-Teilchen, Nanobeads und Marker-Teilchen jeweils mehrere vorgesehen sein.

Gemäß einer Ausführungsart des Verfahrens ist es so, dass
- die erste magnetische Kraft durch einen ersten Magneten aufgebracht wird und/oder
- die zweite magnetische Kraft durch einen zweiten Magneten aufgebracht wird und/oder
- wobei
   o der jeweilige Magnet in das jeweilige Fluid getaucht wird,
   o infolge magnetischer Kräfte sich das jeweilige magnetische Nanobead zu dem jeweiligen Magneten bewegt und anschließend an dem jeweiligen Magneten gehalten ist,
   o sodass Magnet und Nanobead zur Entfernung des Nanobeads gemeinsam aus dem Fluid entnommen werden.

Die genannten magnetischen Kräfte durch einen Magneten zu realisieren, der in das jeweilige Fluid getaucht wird, hat den Vorteil, dass die jeweiligen Nanobeads direkt an dem jeweiligen Magneten gehalten werden. Der Magnet kann anschließend zum Entfernen der Nanobeads aus dem jeweiligen Fluid einfach aus dem Fluid entnommen werden. Die Nanobeads und daran gebundene Prüfsubstanz-Teilchen oder indirekt gebundene Marker-Teilchen werden so ebenfalls einfach aus dem Fluid entnommen. Indem der Magnet nach dem Eintauchen durch das jeweilige Fluid bewegt wird, kann sichergestellt werden, dass wirklich alle Nanobeads in Kontakt mit dem Magneten kommen. So wird sichergestellt, dass möglichst alle potentiell zu entfernenden Nanobeads aus dem Fluid entnommen werden. Ferner sind anschließend die Nanobeads an den Magneten gehalten, der frei von Verunreinigungen ist. So wird die Zuverlässigkeit des Verfahrens erhöht.

Gemäß einer Ausführungsart des Verfahrens ist es so, dass beim Erstellen des zweiten Fluids
- die dritte magnetische Kraft durch einen dritten Magneten aufgebracht wird,
   o der in das Prüffluid getaucht wird,
   o infolge magnetischer Kräfte sich das erste magnetische Nanobead zu dem dritten Magneten bewegt und anschließend an dem dritten Magneten gehalten ist,
   o sodass Magnet und erstes magnetisches Nanobead zur Entfernung des ersten magnetischen Nanobeads gemeinsam aus dem Fluid entnommen werden, und
- die vierte magnetische Kraft durch Umpolung des dritten Magneten erzeugt wird, sodass beim Erstellen der zweiten Fluid an dem Magneten gehaltenes, erstes magnetisches Nanobead abgestoßen wird, wobei zum Einbringen des Nanobeads in die dritte Nanobead-Fluid der erste Magnet zuerst in die dritte Nanobead-Fluid getaucht wird, bevor der Magnet umgepolt wird.

Bei der alternativen Ausführungsart des Verfahrens, bei der das zweite Fluid in mehreren Teilschritten erstellt wird, muss zuerst mit einer dritten magnetischen Kraft das erste magnetische Nanobead aus dem Prüffluid entfernt werden, bevor es anschließend zum Erstellen des zweiten Prüffluids in das dritte Nanobead-Fluid eingebracht wird. Zum Einbringen wird eine vierte magnetische Kraft verwendet. Vorteilhaft ist es, wenn die vierte magnetische Kraft durch eine Umpolung des dritten Magneten geschieht, wozu dieser zum Beispiel als Elektromagnet ausgeführt ist. Das erste magnetische Nanobead ist an dem Magneten gehalten, nachdem dieser in das Gemisch aus Prüffluid und erstem Nanobead-Fluid gehalten wurde. Sobald der dritte Magnet aus dem Gemisch entnommen und in die dritte Nanobead gehalten wird, kann er umgepolt werden, sodass die magnetischen Nanobeads abgestoßen werden. Es sind dann zum Einbringen des ersten magnetischen Nanobeads in das dritte Nanobead-Fluid keine weiteren Magneten oder Hilfsmittel nötig. Es muss lediglich der dritte Magnet in die dritte Nanobead-Fluid gehalten und umgepolt werden. So ist das Verfahren einfach auszuführen.

Gemäß einer Ausführungsart des Verfahrens ist es so, dass beim Erstellen des zweiten Fluid
- die dritte magnetische Kraft durch einen dritten Magneten aufgebracht wird,
   o der in das Prüffluid getaucht wird,
   o infolge magnetischer Kräfte sich das erste magnetische Nanobead zu dem dritten Magneten bewegt und anschließend an dem dritten Magneten gehalten ist,
   o sodass Magnet und erstes magnetisches Nanobead zur Entfernung des ersten magnetischen Nanobeads gemeinsam aus dem Fluid entnommen werden, und
- die vierte magnetische Kraft durch ein von außen auf das dritte Nanobead-Fluid aufgeprägtes Magnetfeld erzeugt wird, wobei die vierte magnetische Kraft erst aufgeprägt wird, nachdem der dritte Magnet mit dem an dem dritten Magneten gehaltenen, ersten magnetischen Nanobead in das dritte Nanobead-Fluid getaucht wurde.

Anstatt, wie zuvor beschrieben, den dritten Magneten umzupolen, ist es auch möglich, die vierte magnetische Kraft durch ein von außen auf das dritte Nanobead-Fluid aufgeprägtes Magnetfeld zu erzeugen. Dazu wird der dritte Magnet mit daran gehaltenem, ersten magnetischen Nanobead in das dritte Nanobead-Fluid getaucht. Anschließend wird die vierte magnetische Kraft erzeugt, z.B. indem ein weiterer, stärkerer Magnet in die Nähe des dritten Nanobead-Fluids gebracht wird. Diese magnetische Kraft ist so stark, dass es das erste magnetische Nanobeads von dem dritten Magneten löst und dieses so auf einfache Weise in das dritte Nanobead-Fluid eingebracht wird, inklusive des eventuell an das erste magnetische Nanobead gebundenen Prüfsubstanz-Teilchens.

Gemäß einer Ausführungsart des Verfahrens ist es so, dass der erste und/oder zweite und/oder dritte Magnet von einer Schutzschicht umschlossen sind, sodass das jeweilige magnetische Nanobead infolge der magnetischen Kräfte an der Schutzschicht gehalten ist.

Wenn die Magnete mit einer Schutzschicht umgegeben sind, braucht zwischen Wiederholungen des Verfahrens lediglich die Schutzschicht gewechselt werden, anstatt dass der gesamte Magnet ausgetauscht werden muss. Hierdurch werden die Kosten für das Verfahren gesenkt und eine Automatisierbarkeit vereinfacht.

Vorteilhaft ist die Kombination dieser Ausführungsart mit der zuvor beschriebenen Ausführungsart, bei der eine vierte Magnetkraft eingesetzt wird. Sofern der dritte Magnet in der ihn umgebenden Schutzschicht beweglich ist und er relativ zu dem Magnetfeld entsprechend orientiert ist, wird er durch die vierte Magnetkraft abgestoßen und in der Schutzschicht bewegt. Auf diese Weise kann die auf die ersten magnetischen Nanobeads wirkende magnetische Kraft des dritten Magneten abgeschwächt werden und eine Einbringung des ersten magnetischen Nanobeads in das dritte Nanobead-Fluid ist vereinfacht.

Gemäß einer Ausführungsart des Verfahrens ist es so, dass die Anzahl der ursprünglich in dem Prüffluid vorhandenen Prüfsubstanz-Teilchen bestimmt wird, indem
- eine bekannte Zahl an nicht-magnetischen Nanobeads mit jeweils bekannter Anzahl an komplementären Bindungsstellen erster und dritter Art verwendet wird,
   o wobei die Anzahl der an den nicht-magnetischen Nanobeads insgesamt vorhandenen komplementären Bindungsstellen erster Art größer als die Anzahl der Prüfsubstanz-Teilchen ist
   o und wobei die Anzahl der an den nicht-magnetischen Nanobeads insgesamt vorhandenen komplementären Bindungsstellen dritter Art kleiner als die Anzahl der Marker-Teilchen ist, und
- die Differenz zwischen den am Ende in dem dritten Fluid verbliebenen Marker-Teilchen und den zu Beginn vorhandenen Marker-Teilchen bestimmt wird,
- die Anzahl der ursprünglich vorhandenen Prüfsubstanz-Teilchen mittels der Differenz der Marker-Teilchen unter Kenntnis der Anzahl der nicht-magnetischen Nanobeads berechnet wird.

Mit dem Verfahren ist es nicht nur möglich, das bloße Vorhandensein einer Prüfsubstanz nachzuweisen. Es ist auch durch die beschriebene Ausführungsart möglich, mit dem Verfahren die Anzahl der vorhandenen Prüfsubstanz-Teilchen zu quantifizieren. Dazu ist es nötig zu wissen, wie viele komplementäre Bindungsstellen erster Art und dritter Art das nicht-magnetische Nanobead aufweist. Dies ist durch eine entsprechende Herstellung des Nanobeads möglich. Bei der Verwendung mehrere nicht-magnetischer Nanobeads kann dafür gesorgt werden, dass alle nicht-magnetischen Nanobead zumindest näherungsweise gleich viele der jeweiligen Bindungsstellen aufweisen, indem sichergestellt wird, dass alle nicht-magnetischen Nanobeads gleichgroß sind, z.B. indem eine Aufreinigung handelsüblicher Nanobead-Fluide stattfindet.

Die komplementären Bindungsstellen erster Art binden die Prüfsubstanz-Teilchen. Sind mehrere von diesen Bindungsstellen an einem nicht-magnetischen Nanobead vorhanden, kann ein nicht-magnetisches Nanobead mehrere Prüfsubstanz-Teilchen binden. Gleiches gilt auch für das Marker-Teilchen, welches durch komplementäre Bindungsstellen dritter Art an das nicht-magnetische Nanobead gebunden wird. Das Verhältnis von komplementären Bindungsstellen erster Art zu komplementären Bindungsstellen dritter Art an dem nicht-magnetischen Nanobead gibt an, wie viel weniger Marker-Teilchen in dem dritten Fluid verbleiben, wenn ein nicht-magnetisches Nanobead aus dem zweiten Fluid entfernt wurde, was wiederum einer Entfernung der entsprechenden Anzahl an Prüfsubstanz-Teilchen entspricht. Durch eine Messung der Veränderung der Anzahl der Marker-Teilchen kann unter Kenntnis des Verhältnisses auf die Anzahl der Prüfsubstanz-Teilchen geschlossen werden, die zusammen mit nicht-magnetischen Nanobeads aus dem zweiten Fluid entfernt wurden. Die Messung der Veränderung der Anzahl der Marker-Teilchen kann durch eine Veränderung der Konzentration der Marker-Teilchen bestimmt werden, welche z.B. durch die Stärke des Fluoreszierens des mit Marker-Teilchen versetzten dritten Fluids im Vergleich zur Stärke des Fluoreszierens des Marker-Fluids unter Kenntnis der jeweiligen Flüssigkeitsmengen bestimmt wird.

Wichtig ist dabei, dass mehr komplementäre Bindungsstellen erster Art als Prüfsubstanz-Teilchen und mehr Marker-Teilchen als komplementäre Bindungsstellen dritter Art aller eingesetzten nicht-magnetischen Nanobeads vorhanden sind. Andernfalls können nicht alle Prüfsubstanz-Teilchen gebunden werden und nicht an jedem nicht-magnetischen Nanobead die entsprechende Anzahl an Marker-Teilchen gebunden werden, weil bereits alle Marker-Teilchen gebunden sein könnten, obwohl noch nicht-magnetische Nanobeads in dem dritten Fluid vorhanden sind.

Gemäß einer Ausführungsart des Verfahrens ist es so, dass das Behältnis, in dem das zweite Fluid erstellt wird, nach unten konisch zuläuft.

Die Nanobeads haben in der Regel eine höhere Dichte als das Fluid, in dem sie enthalten sind. Folglich sammeln sich die Nanobeads ohne eine entsprechende Mischung am Boden der Flüssigkeit, sodass an dieser Stelle alle an dem Verfahren beteiligten Nanobeads zusammenkommen. So ist sichergestellt, dass alle entsprechenden Nanobeads eine Bindung ausbilden können.

Die Erfindung wird nachfolgend anhand von zwei Ausführungsbeispielen näher erläutert, die in den anliegenden Zeichnungen dargestellt sind. In den Zeichnungen zeigen:

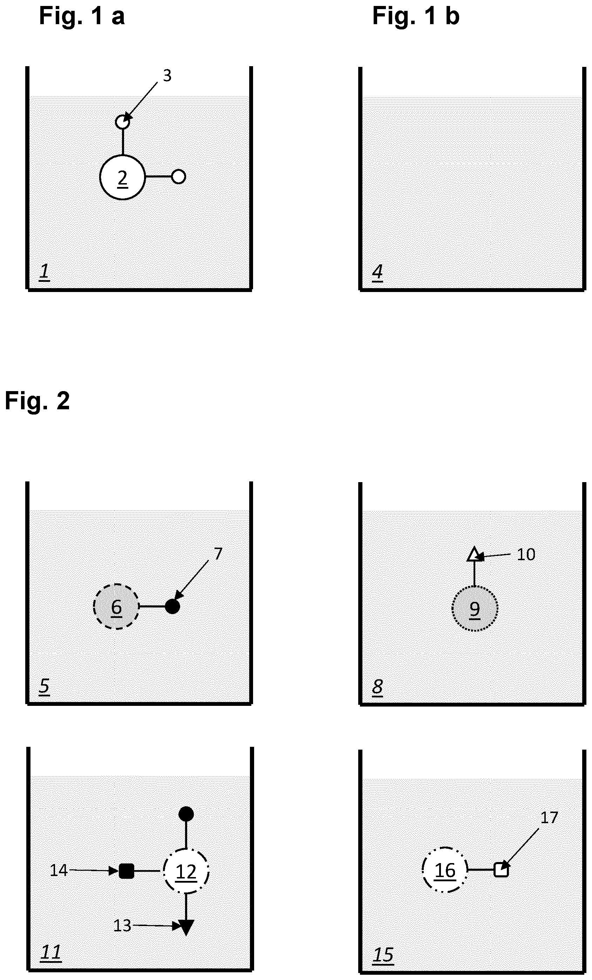

- Fig. 1 a: das Prüffluid im Fall des Vorhandenseins des Prüfsubstanz-Teilchens;
- Fig. 1 b: das Prüffluid im Fall des Nichtvorhandenseins des Prüfsubstanz-Teilchens;
- Fig. 2: die drei Nanobead-Fluide und das Marker-Fluid;
- Fig. 3 a: Erstellen des zweiten Fluids im Fall des Vorhandenseins des Prüfsubstanz-Teilchens;

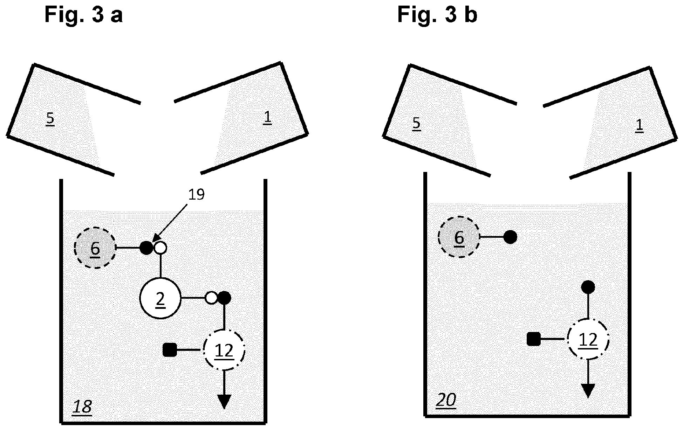

- Fig. 3 b: Erstellen des zweiten Fluids im Fall des Nichtvorhandenseins des Prüfsubstanz-Teilchens;

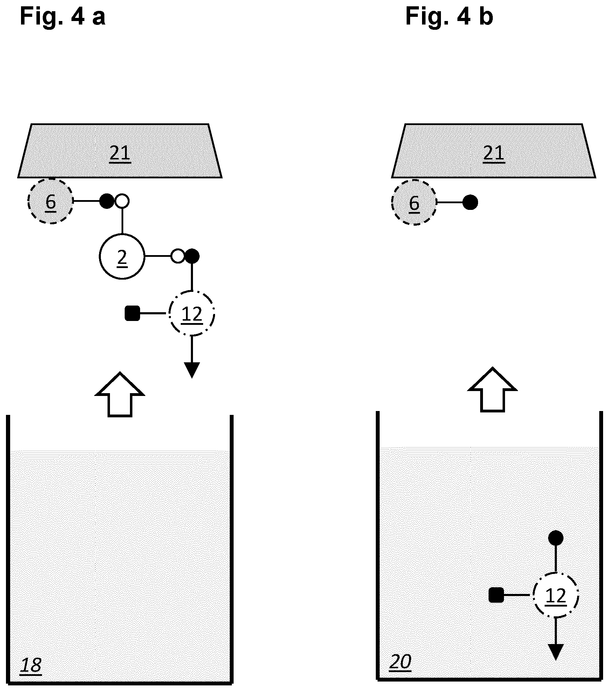

- Fig. 4 a: Aufbringen der ersten Magnetkraft im Fall des Vorhandenseins des Prüfsubstanz-Teilchens;
- Fig. 4 b: Aufbringen der ersten Magnetkraft des Nichtvorhandenseins des Prüfsubstanz-Teilchens;

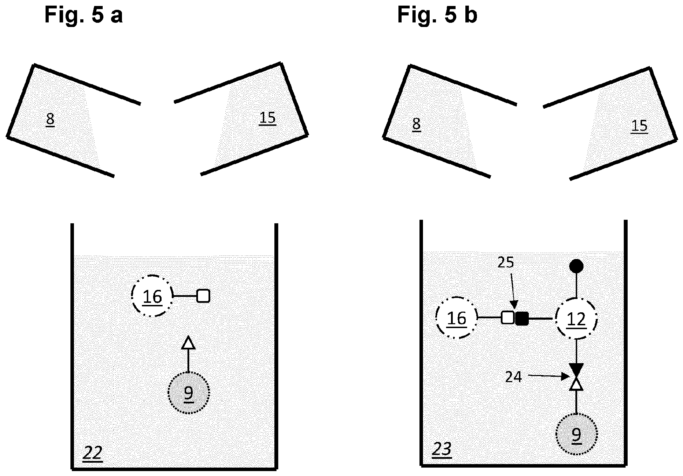

- Fig. 5 a: Erstellen des dritten Fluids im Fall des Vorhandenseins des Prüfsubstanz-Teilchens;
- Fig. 5 b: Erstellen des dritten Fluids im Fall des Nichtvorhandenseins des Prüfsubstanz-Teilchens;

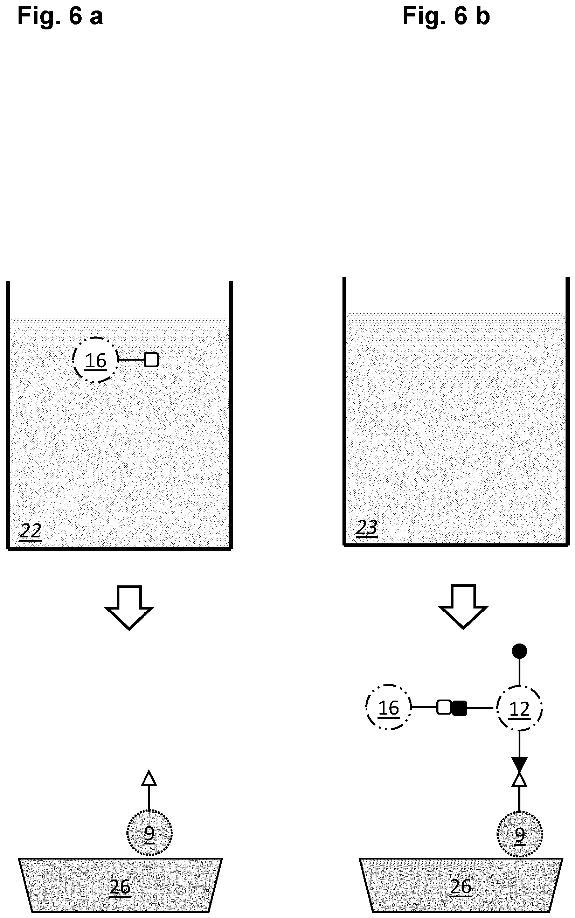

- Fig. 6 a: Aufbringen der zweiten Magnetkraft im Fall des Vorhandenseins des Prüfsubstanz-Teilchens;
- Fig. 6 b: Aufbringen der zweiten Magnetkraft im Fall des Nichtvorhandenseins des Prüfsubstanz-Teilchens;

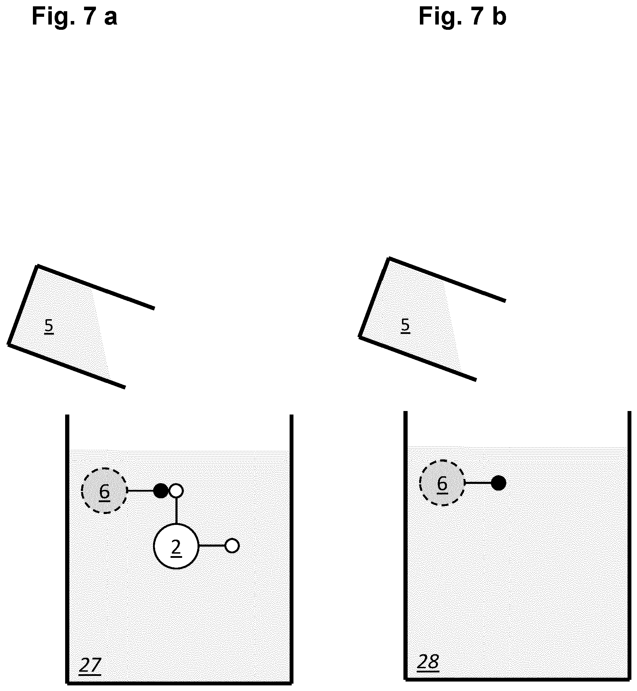

- Fig. 7 a: Vermischen des Prüffluids und des ersten Nanobead-Fluids im Fall des Vorhandenseins des Prüfsubstanz-Teilchens gemäß einer alternativen Ausführungsart;
- Fig. 7 b: Vermischen des Prüffluids und des ersten Nanobead-Fluids im Fall des Nichtvorhandenseins des Prüfsubstanz-Teilchens gemäß einer alternativen Ausführungsart;

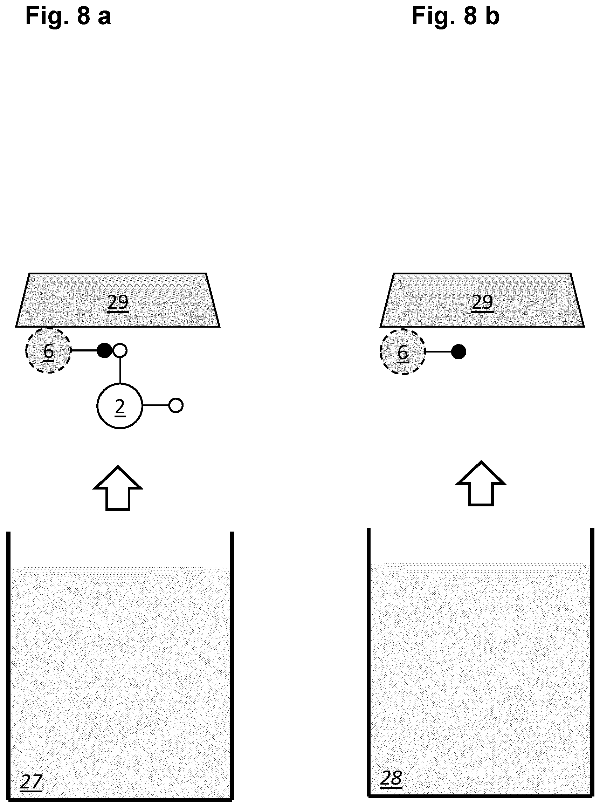

- Fig. 8 a: Aufbringen der ersten magnetischen Kraft im Fall des Vorhandenseins der Prüfsubstanz-Teilchens gemäß einer alternativen Ausführungsart;
- Fig. 8 b: Aufbringen der ersten magnetischen Kraft im Fall des Nichtvorhandenseins der Prüfsubstanz-Teilchens gemäß einer alternativen Ausführungsart;

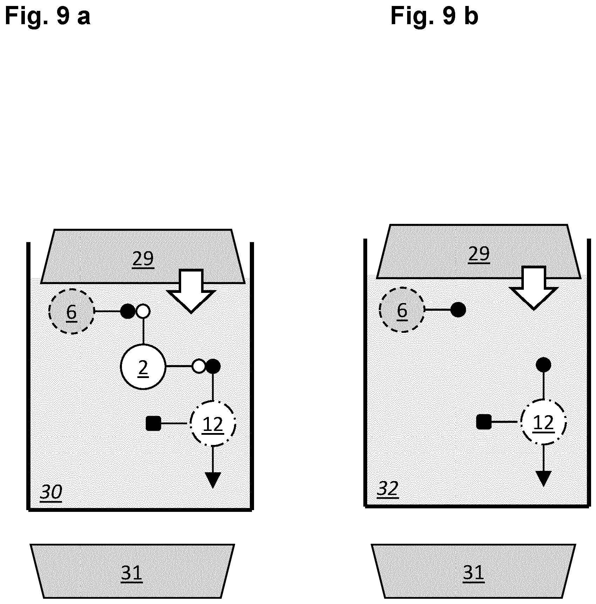

- Fig. 9 a: Erstellen des zweiten Fluids im Fall des Vorhandenseins des Prüfsubstanz-Teilchens gemäß einer alternativen Ausführungsart;
- Fig. 9 b: Erstellen des zweiten Fluids im Fall des Nichtvorhandenseins des Prüfsubstanz-Teilchens gemäß einer alternativen Ausführungsart.

Fig. 1 a zeigt das Prüffluid 1, welche auch als erstes Fluid bezeichnet ist, bei Vorhandensein des Prüfsubstanz-Teilchens 2. Das Prüfsubstanz-Teilchen 2 weist eine Bindungsstelle erster Art 3 auf. Fig. 1 b zeigt das Prüffluid 4 bei Nichtvorhandensein der Prüfsubstanz-Teilchens.

Fig. 2 zeigt das erste Nanobead-Fluid 5, in welchem das erste magnetische Nanobead 6 enthalten ist. Das erste magnetische Nanobead 6 weist eine komplementäre Bindungsstelle erster Art 7 auf. Das zweite Nanobead-Fluid 8 weist ein zweites magnetisches Nanobead 9 auf, das über eine Bindungsstelle zweiter Art 10 verfügt. Das dritte Nanobead-Fluid 11 weist ein nicht-magnetisches Nanobead 12 auf, das neben einer komplementären Bindungsstelle erster Art 7 auch über eine komplementäre Bindungsstelle zweiter Art 13 und eine komplementäre Bindungsstelle dritter Art 14 verfügt. Das Marker-Fluid 15 weist ein Marker-Teilchen 16 auf, das über eine Bindungsstelle dritter Art 17 verfügt.

Fig. 3 a zeigt im Fall des ursprünglichen Vorhandenseins des Prüfsubstanz-Teilchens 2, wie das zweite Fluid 18 erstellt wird, indem das erste Nanobead-Fluid 5 und das Prüfsubstanz-Fluid 1 mit dem dritten Nanobead-Fluid 11 vermischt werden. Das Prüfsubstanz-Teilchen 2 bildet mit dem ersten magnetischen Nanobead 6 und dem nicht-magnetischen Nanobead 12 jeweils eine Bindung erster Art 19 aus. Fig. 3 b zeigt das zweite Fluid 20 für den Fall, dass das Prüfsubstanz-Teilchen nicht in dem Prüffluid vorhanden war. Aufgrund des Nichtvorhandenseins des Prüfsubstanz-Teilchens besteht auch keine indirekte Verbindung zwischen dem zweiten magnetischen Nanobead 6 und dem nicht-magnetischen Nanobead 12.

Fig. 4 a zeigt im Fall des ursprünglichen Vorhandenseins des Prüfsubstanz-Teilchens 2, wie das erste magnetische Nanobead 6 und das daran gebundene Prüfsubstanz-Teilchen 2 und das daran gebundene nicht-magnetische Nanobead 12 aus dem zweiten Fluid 18 mit der durch den ersten Magneten 21 erzeugten ersten Magnetkraft entfernt werden. Fig. 4 b zeigt, dass im Fall des ursprünglichen Nichtvorhandenseins des Prüfsubstanz-Teilchens 2 das nicht-magnetische Nanobead in dem zweiten Fluid verbleibt, weil es nicht indirekt über die Prüfsubstanz an das erste magnetische Nanobead 6 gebunden ist. Das erste magnetische Nanobead 6 wird in diesem Fall jedoch genauso wie beim ursprünglichen Vorhandensein des Prüfsubstanz-Teilchens aus dem zweiten Fluid durch die von dem ersten Magneten 21 aufgebrachte ersten Magnetkraft entfernt.

Fig. 5 a zeigt im Fall des ursprünglichen Vorhandenseins des Prüfsubstanzteilchens, wie das dritte Fluid 22 erstellt wird, in dem das zweite Nanobeadfluid 8 und didase Marker-Fluid 15 in das zweite Fluid 18 von Fig. 4 a eingemischt werden. In dem dritten Fluid 22 sind folglich das zweite magnetische Nanobead 9 und das Marker-Teilchen 16 vorhanden. Fig. 5 b zeigt denselben Vorgang für den Fall des ursprünglichen Nichtvorhandenseins des Prüfsubstanz-Teilchens. In diesem Fall verblieb, wie zuvor anhand von Fig. 4 b erläutert, das nicht-magnetische Nanobead 12 in dem zweiten Fluid 20. Dieses ist nun in dem dritten Fluid 23 vorhanden und bildet mit dem zweiten magnetischen Nanobead 9 eine Bindung zweiter Art 24 aus und mit dem Marker-Teilchen 16 eine Bindung dritter Art 25 aus.

Fig. 6 a zeigt im Fall des ursprünglichen Vorhandenseins des Prüfsubstanz-Teilchens, wie durch die von dem zweiten Magneten 26 erzeugte Magnetkraft das zweite magnetische Nanobead 9 aus dem dritten Fluid 22 entfernt wird. Das Marker-Teilchen 16 verbleibt in dem dritten Fluid 22. Fig. 6 b zeigt denselben Vorgang für den Fall des ursprünglichen Nichtvorhandenseins des Prüfsubstanz-Teilchens. Dadurch, dass in diesem Fall das nicht-magnetische Nanobead 12 in dem dritten Fluid 23 vorhanden war, wird das Marker-Teilchen 16 durch die von dem zweiten Magneten 26 erzeugte Magnetkraft aus dem dritten Fluid 23 entfernt, weil das Marker-Teilchen 16 indirekt über das nicht-magnetische Nanobead 12 an das zweite magnetische Nanobead 9 gebunden ist.

Beim Vergleich des dritten Fluids 22 in Fig. 6 a und dem dritten Fluid 23 in Fig. 6 b fällt auf, dass lediglich im Fall des ursprünglichen Vorhandenseins des Prüfsubstanz-Teilchens das Marker-Teilchen 16 in dem dritten Fluid 22 verbleibt. Das Marker-Teilchen verändert die physikalischen Eigenschaften des Fluids 22 im Vergleich zu dem Fluid 23, sodass anhand dieses Unterschieds leicht ersichtlich ist, ob das Prüfsubstanz-Teilchen ursprünglich vorhanden war. Die Veränderung der physikalischen Eigenschaften kann beispielsweise durch ein Fluoreszieren hervorgerufen sein, welches mit entsprechender Analytik erfasst werden kann.

Fig. 7 a bis 9 b zeigen eine alternative Ausführungsart zur Erstellung des zweiten Fluids. Die Erstellung des zweiten Fluids erfolgt nicht, wie in Fig. 3 a und Fig. 3 b gezeigt, in einem Schritt, sondern in drei Teilschritten.

Fig. 7 a zeigt den ersten Teilschritt für den Fall des ursprünglichen Vorhandenseins des Prüfsubstanz-Teilchens 2 in dem Prüffluid. Bei diesem Teilschritt entsteht das Gemisch 27 durch das Einmischen des ersten Nanobead-Fluids 5 in das Prüffluid. Anschließend bilden in dem Gemisch 27 das Prüfsubstanz-Teilchen 2 und das erste magnetische Nanobead 6 eine Bindung aus. Fig. 7 zeigt denselben Teilschritt für den Fall des ursprünglichen Nichtvorhandenseins des Prüfsubstanz-Teilchens. Folglich befindet sich in dem Gemisch 28 aus erstem Nanobeadfluid 5 und Prüffluid nur das erste magnetische Nanobead 6.

Fig. 8 a zeigt den nächsten Teilschritt für den Fall des ursprünglichen Vorhandenseins des Prüfsubstanz-Teilchens 2. In diesem Teilschritt wird durch die von dem dritten Magneten 29 aufgebrachte magnetische Kraft das erste magnetische Nanobead 6 zusammen mit dem an es gebundenen Prüfsubstanz-Teilchen 2 aus dem Gemisch 27 entfernt. Fig. 8 b zeigt denselben Schritt für den Fall des ursprünglichen Nichtvorhandenseins der Prüfsubstanz-Teilchens. In diesem Fall wird nur das erste magnetische Nanobead aus dem Gemisch 28 entfernt.

Fig. 9 a zeigt den nächsten Teilschritt für den Fall des ursprünglichen Vorhandenseins des Prüfsubstanz-Teilchens 2. Zur Erstellung des zweiten Fluid 30 wird das Prüfsubstanz-Teilchens 2 gemeinsam mit dem ersten magnetischen Nanobead 6 durch die von dem vierten Magneten 31 erzeugten vierte magnetische Kraft in das dritte Nanobead-Fluid 11 eingebracht. Dadurch wird das dritte Nanobead-Fluid 11 zum zweiten Fluid 30. Dazu wird der dritte Magnet 29 in das dritte Nanobead-Fluid 11 eingebracht und dann die vierte magnetische Kraft aufgebracht, indem der vierte Magnet 31 unter das Gefäß des dritten Nanobead-Fluids 11 gehalten wird. Die durch den vierten Magneten 31 aufgebrachte vierte magnetische Kraft ist größer als die dritte magnetische Kraft, sodass das an dem dritten Magneten 29 gehaltene zweite magnetische Nanobead 6 inklusive des an ihm gebundenen Prüfsubstanz-Teilchens 2 in das dritte Nanobead-Fluid 11 eingebracht werden. Da das dritte Nanobead-Fluid 11 das nicht-magnetische Nanobead 12 bereits enthielt, enthält das zweite Fluid 30 nun ebenfalls das nicht-magnetische Nanobead 12. Das Prüfsubstanz-Teilchen 2 und das nicht-magnetische Nanobead 12 bilden nach dem Einbringen in das dritte Nanobead-Fluid eine Bindung aus, sodass das erste magnetische Nanobead 6 indirekt über das Prüfsubstanz-Teilchen 2 an das nicht-magnetische Nanobead 12 gebunden ist. Fig. 9 b zeigt denselben Teilschritt für den Fall des ursprünglichen Nichtvorhandenseins des Prüfsubstanz-Teilchens. In diesem Fall fand keine Bindung des Prüfsubstanz-Teilchens an das erste magnetische Nanobead 6 statt, sodass durch die von dem vierten Magneten 31 erzeugte vierte Magnetkraft nur das erste magnetische Nanobead 6 in das dritte Nanobead-Fluid zum Erstellen des zweiten Fluids 32 eingebracht wird. Ohne die Prüfsubstanz kann das erste magnetische Nanobead 6 auch nicht indirekt an das nicht-magnetische Nanobead 12 binden.

Das zweite Fluid 30 aus Fig. 9 a entspricht dem zweiten Fluid 18 aus Fig. 3 a, während das zweite Fluid 32 aus Fig. 9 b dem zweiten Fluid 20 aus Fig. 3 b entspricht.

Durch die alternative Ausführungsart gemäß Fig. 7 a bis Fig. 9 b kommt das nicht-magnetische Nanobead 12 nicht mit dem Prüffluid in Kontakt. Dadurch wird verhindert, dass sogenannte Matrix-Effekte entstehen, welche eine unerwünschte Reaktion des nicht-magnetischen Nanobeads mit Verunreinigungen bezeichnen.

### BEZUGSZEICHEN

- 1: Prüffluid bei Vorhandensein des Prüfsubstanz-Teilchens
- 2: Prüfsubstanz-Teilchen
- 3: Bindungstelle erster Art
- 4: Prüffluid bei Nichtvorhandensein des Prüfsubstanz-Teilchens
- 5: Erstes Nanobead-Fluid
- 6: Erstes magnetisches Nanobead
- 7: Komplementäre Bindungsstelle erster Art
- 8: Zweites Nanobead-Fluid
- 9: Zweites magnetisches Nanobead
- 10: Bindungsstelle zweiter Art
- 11: Drittes Nanobead-Fluid
- 12: Nicht-magnetisches Nanobead
- 13: Komplementäre Bindungsstelle zweiter Art
- 14: Komplementäre Bindungsstelle dritter Art
- 15: Marker-Fluid
- 16: Marker-Teilchen
- 17: Bindungsstelle dritter Art
- 18: Zweites Fluid bei Vorhandensein des Prüfsubstanz-Teilchens
- 19: Bindung erster Art
- 20: Zweites Fluid bei Nichtvorhandensein des Prüfsubstanz-Teilchens
- 21: Erster Magnet
- 22: Drittes Fluid bei Vorhandensein des Prüfsubstanz-Teilchens
- 23: Drittes Fluid bei Nichtvorhandensein des Prüfsubstanz-Teilchens
- 24: Bindung zweiter Art
- 25: Bindung dritter Art
- 26: Zweiter Magnet
- 27: Gemisch aus Prüfsubstanz-Fluid und erstem Nanobead-Fluid bei Vorhandensein des Prüfsubstanz-Teilchens
- 28: Gemisch aus Prüfsubstanz-Fluid und erstem Nanobead-Fluid bei Nichtvorhandensein des Prüfsubstanz-Teilchens
- 29: Dritter Magnet
- 30: Zweites Fluid bei Vorhandensein des Prüfsubstanz-Teilchens (Verfahrensalternative)
- 31: Vierter Magnet
- 32: Zweites Fluid bei Nichtvorhandensein des Prüfsubstanz-Teilchens (Verfahrensalternative)

## Patentansprüche

1. Verfahren zum direkten Nachweis des Vorhandenseins einer Prüfsubstanz in einem Prüffluid umfassend die folgenden Schritte:
i. Bereitstellen eines ersten Fluids, dem Prüffluid, in dem in einem ersten Fall ein Prüfsubstanz-Teilchen vorhanden ist und in einem zweiten Fall das Prüfsubstanz-Teilchen nicht vorhanden ist,
o wobei das Prüfsubstanz-Teilchen Bindungsstellen erster Art aufweist,
ii. Bereitstellen von
o eines ersten Nanobead-Fluids, das ein erstes magnetisches Nanobead mit einer komplementären Bindungsstelle erster Art enthält,
o eines zweiten Nanobead-Fluids, das ein zweites magnetisches Nanobead mit einer Bindungsstelle zweiter Art enthält,
o eines dritten Nanobead-Fluids, das ein nicht-magnetisches Nanobead mit einer komplementären Bindungsstelle erster Art, einer komplementären Bindungsstelle zweiter Art und einer komplementären Bindungsstelle dritter Art enthält,
o eines Marker-Fluids, das ein Marker-Teilchen mit einer Bindungsstelle dritter Art enhält,
o wobei bei Vorhandensein entsprechender Bindungspartner in demselben Fluid
▪ eine Bindungsstelle erster Art eines Prüfsubstanz-Teilchens mit einer komplementären Bindungsstelle erster Art eines magnetischen Nanobeads erster Art und/oder eines nicht-magnetischen Nanobeads eine Bindung erster Art ausbildet,
▪ eine Bindungsstelle zweiter Art eines magnetischen Nanobeads zweiter Art mit einer komplementären Bindungsstelle zweiter Art eines nicht-magnetischen Nanobeads eine Bindung zweiter Art ausbildet,
▪ eine Bindungsstellen dritter Art eines Marker-Teilchens mit einer komplementären Bindungsstelle dritter Art eines nicht-magnetischen Nanobeads eine Bindung dritter Art ausbildet,
iii. Erstellen eines zweiten Fluids,
o indem das Prüffluid mit dem ersten Nanobead-Fluid vermischt wird und zumindest das erste magnetische Nanobead des resultierenden Fluids mit dem dritten Nanobead-Fluid vermischt wird, wonach das resultierende Fluid als zweites Fluid bezeichnet ist,
iv. Entfernen des ersten magnetischen Nanobeads aus des zweiten Fluids mittels einer ersten magnetischen Kraft,
o wobei im ersten Fall das an das erste magnetische Nanobead gebundene Prüfsubstanz-Teilchen und das an das Prüfsubstanz-Teilchen gebundene, nicht-magnetische Nanobead ebenfalls aus dem zweiten Fluid entfernt werden
∘ oder im zweiten Fall das nicht-magnetische Nanobead in dem zweiten Fluid verbleibt,
v. Erstellen eines dritten Fluids, indem
o das zweite Nanobead-Fluid und das Marker-Fluid in das zweite Fluid eingemischt werden, wonach das Gemisch als drittes Fluid bezeichnet ist,
vi. Entfernen des zweiten magnetischen Nanobeads aus dem dritten Fluid mittels einer zweiten magnetischen Kraft,
o wobei im ersten Fall aufgrund des Nichtvorhandenseins des nicht-magnetischen Nanobeads in dam dritten Fluid das Marker-Teilchen nicht indirekt an das zweite magnetische Nanobeads gebunden ist, sodass es nach dem Entfernen des zweiten magnetischen Nanobeads in dem dritten Fluid verbleibt, oder
o wobei im zweiten Fall das nicht-magnetische Nanobead an das zweite magnetische Nanobead gebunden ist und das Marker-Teilchen an das nicht-magnetische Nanobead gebunden ist, sodass das Marker-Teilchen zusammen mit dem zweiten magnetischen Nanobead und dem nicht-magnetischen MarkerTeilchen aus dem dritten Fluid entfernt wird.

2. Verfahren nach Anspruch 1, bei dem zum Erstellen des zweiten Fluids das dritte Nanobead-Fluid mit dem Prüffluid und mit dem ersten Nanobead-Fluid vermischt wird.

3. Verfahren nach Anspruch 1, bei dem zum Erstellen des zweiten Fluids
• als erstes das erste Nanobead-Fluid mit dem Prüffluid vermischt wird,
• als zweites das erste magnetische Nanobead durch eine dritte magnetische Kraft aus dem Prüffluid entfernt wird und
• als drittes das aus dem Prüffluid entfernte erste magnetische Nanobead durch eine vierte magnetische Kraft in das dritte Nanobead-Fluid eingebracht wird.

4. Verfahren nach einem der Ansprüche 1 bis 3, bei dem
• das Prüfsubstanzteilchen zwei oder mehr Bindungsstellen erster Art aufweist,
• das erste magnetische Nanobead eine einzelne oder mehr komplementäre Bindungsstellen erster Art aufweist,
• das zweite magnetische Nanobead eine einzelne oder mehr Bindungsstellen zweiter Art aufweist,
• das nicht-magnetische Nanobead eine einzelne oder mehr komplementäre Bindungsstellen erster Art, eine einzelne oder mehr komplementäre Bindungsstellen zweiter Art, eine einzelne oder mehr komplementäre Bindungsstellen dritter Art aufweist sowie
• das Marker Teilchen eine einzelne oder mehre Bindungsstellen dritter Art aufweist.

5. Verfahren nach einem der Ansprüche 1 bis 4, bei dem
• das Prüffluid ein einzelnes oder mehrere Prüfsubstanz-Teilchen enthält und/oder
• das erste Nanobead-Fluid ein einzelnes oder mehrere erste magnetische Nanobeads enthält und/oder
• das zweite Nanobead-Fluid ein einzelnes oder mehrere zweites magnetische Nanobeads enthält und/oder
• das dritte Nanobead-Fluid ein einzelnes oder mehrere nicht-magnetische Nanobeads enthält und/oder
• das erste Marker-Fluid ein einzelnes oder mehrere Marker-Teilchen enthält werden.

6. Verfahren nach einem der Ansprüche 1 bis 5, bei dem die Bindung erster Art eine Antikörper-Antigen-Bindung oder eine Oligonucleotid-Bindung oder eine Lipid-Bindung oder eine Glucose-Bindung ist.

7. Verfahren nach einem der Ansprüche 1 bis 6, bei dem die Bindung zweiter Art eine Antikörper-Antigen-Bindung oder eine Oligonucleotid-Bindung oder eine Lipid-Bindung oder eine Glucose-Bindung ist.

8. Verfahren nach einem der Ansprüche 1 bis 7, bei dem die Bindung dritter Art eine Antikörper-Antigen-Bindung oder eine Oligonucleotid-Bindung oder eine Lipid-Bindung oder eine Glucose-Bindung ist.

9. Verfahren nach einem der Ansprüche 1 bis 9, bei dem das Marker-Teilchen ein farbiges Teilchen, ein leuchtendes Teilchen, ein fluoreszierendes Teilchen, ein die elektrische Leitfähigkeit einer Flüssigkeit beeinflussendes Teilchen oder ein radioaktives Teilchen ist.

10. Verfahren nach einem der Ansprüche 1 bis 10, bei dem
• sich in dem Prüffluid ein zusätzliches Prüfsubstanz-Teilchen mit einer alternativen Bindungsstelle erster Art befindet,
• ein zusätzliches erstes Nanobead-Fluid bereitgestellt wird, das ein alternatives erstes Nanobead mit einer alternativen komplementären Bindungsstelle erster Art enthält, und
• ein zusätzliches drittes Nanobead-Fluid bereitgestellt wird, das ein alternatives nicht-magnetisches Nanobead mit einer alternativen komplementären Bindungsstelle erster Art und einer alternativen komplementären Bindungsstelle dritter Art enhält, und
• ein zusätzliches Marker-Fluid bereitgestellt wird, das ein alternatives Marker-Teilchen mit einer alternativen Bindungsstelle dritter Art enthält,
• wobei das zusätzliche erste Nanobead-Fluid, das zusätzliche dritte Nanobead-Fluid und das zusätzliche Marker-Fluid in dem Verfahren bei denselben Schritten und in derselben Weise wie das erste Nanobead-Fluid, das dritte Nanobead-Fluid bzw. das Marker-Fluid eingesetzt werden.

11. Verfahren nach Anspruch 10, bei dem
• das Prüffluid ein einzelnes oder mehrere zusätzliche Prüfsubstanz-Teilchen enthält, und/oder
• das erste zusätzliche Nanobead-Fluid ein einzelnes oder mehrere alternative erste Nanobeads enthält und/oder
• das dritte zusätzliche Nanobead-Fluid ein einzelnes oder mehrere alternative dritte Nanobeads enthält und/oder
• das zusätzliche Marker-Fluid ein einzelnes oder mehrere alternative Marker-Teilchen enthält.

12. Verfahren nach einem der Ansprüche 1 bis 11, bei dem
• die erste magnetische Kraft durch einen ersten Magneten aufgebracht wird und/oder
• die zweite magnetische Kraft durch einen zweiten Magneten aufgebracht wird und/oder
• wobei
o der jeweilige Magnet in das jeweilige Fluid getaucht wird,
o infolge magnetischer Kräfte sich das jeweilige magnetische Nanobead zu dem jeweiligen Magneten bewegt und anschließend an dem jeweiligen Magneten gehalten ist,
o sodass Magnet und Nanobead zur Entfernung des Nanobeads gemeinsam aus dem Fluid entnommen werden.

13. Verfahren nach Anspruch 3, bei dem beim Erstellen des zweiten Fluids
• die dritte magnetische Kraft durch einen dritten Magneten aufgebracht wird,
o der in das Prüffluid getaucht wird,
o infolge magnetischer Kräfte sich das erste magnetische Nanobead zu dem dritten Magneten bewegt und anschließend an dem dritten Magneten gehalten ist,
o sodass Magnet und erstes magnetisches Nanobead zur Entfernung des ersten magnetischen Nanobeads gemeinsam aus dem Fluid entnommen werden, und
• die vierte magnetische Kraft durch Umpolung des dritten Magneten erzeugt wird, sodass beim Erstellen des zweiten Fluids an dem Magneten gehaltenes, erstes magnetisches Nanobead abgestoßen wird, wobei zum Einbringen des Nanobeads in das dritte Nanobead-Fluid der erste Magnet zuerst in das dritte Nanobead-Fluid getaucht wird, bevor der Magnet umgepolt wird.

14. Verfahren nach Anspruch 3, bei dem beim Erstellen des zweiten Fluids
• die dritte magnetische Kraft durch einen dritten Magneten aufgebracht wird,
o der in das Prüffluid getaucht wird,
o infolge magnetischer Kräfte sich das erste magnetische Nanobead zu dem dritten Magneten bewegt und anschließend an dem dritten Magneten gehalten ist,
o sodass Magnet und erstes magnetisches Nanobead zur Entfernung des ersten magnetischen Nanobeads gemeinsam aus dem Fluid entnommen werden, und
• die vierte magnetische Kraft durch ein von außen auf das dritte Nanobead-Fluid aufgeprägtes Magnetfeld erzeugt wird, wobei die vierte magnetische Kraft erst aufgeprägt wird, nachdem der dritte Magnet mit dem an dem dritten Magneten gehaltenen, ersten magnetischen Nanobead in das dritte Nanobead-Fluid getaucht wurde.

15. Verfahren nach einem der Ansprüche 12 bis 14, bei dem der erste und/oder zweite und/oder dritte Magnet von einer Schutzschicht umschlossen sind, sodass das jeweilige magnetische Nanobead infolge der magnetischen Kräfte an der Schutzschicht gehalten ist.

16. Verfahren nach einem der Ansprüche 1 bis 15, bei dem die Anzahl der ursprünglich in das Prüffluid vorhandenen Prüfsubstanz-Teilchen bestimmt wird, indem
• eine bekannte Zahl an nicht-magnetischen Nanobeads mit jeweils bekannter Anzahl an komplementären Bindungsstellen erster und dritter Art verwendet wird,
o wobei die Anzahl der an den nicht-magnetischen Nanobeads insgesamt vorhandenen komplementären Bindungsstellen erster Art größer als die Anzahl der Prüfsubstanz-Teilchen ist
o und wobei die Anzahl der an den nicht-magnetischen Nanobeads insgesamt vorhandenen komplementären Bindungsstellen dritter Art kleiner als die Anzahl der Marker-Teilchen ist, und
• die Differenz zwischen den am Ende in dem dritten Fluid verbliebenen Marker-Teilchen und den zu Beginn vorhandenen Marker-Teilchen bestimmt wird,
• die Anzahl der ursprünglich vorhandenen Prüfsubstanzen mittels der Differenz der Marker-Teilchen unter Kenntnis der Anzahl der nicht-magnetischen Nanobeads berechnet wird.

17. Verfahren nach einem der Ansprüche 1 bis 16, bei dem das Behältnis, in dem das zweite Fluid erstellt wird, nach unten konisch zuläuft.

18. Verfahren nach einem der Ansprüche 1 bis 17, das zum Nachweis der Prüfsubstanz in dem Prüffluid eingesetzt wird, wenn die Anzahl der Prüfsubstanz-Teilchen im femto- und/oder atto- und/oder zeptomoleren Bereich liegt.

19. Verfahren nach einem der Ansprüche 1 bis 18, das für Single-Molecule-Counting eingesetzt wird.

## Claims

1. A method for directly detecting the presence of a test substance in a test fluid, comprising the following steps:
i. providing a first fluid, the test fluid, in which a test substance particle is present in a first case and the test substance particle is not present in a second case,
o wherein the test substance particle has binding sites of a first type,
ii. providing
o a first nanobead fluid containing a first magnetic nanobead with a complementary binding site of a first type,
o a second nanobead fluid containing a second magnetic nanobead with a binding site of a second type,
o a third nanobead fluid containing a non-magnetic nanobead with a complementary binding site of a first type, a complementary binding site of a second type, and a complementary binding site of a third type,
o a marker fluid containing a marker particle with a binding site of a third type,
o wherein, in the presence of corresponding binding partners in the same fluid,
• a binding site of a first type of a test substance particle forms a binding of a first type with a complementary binding site of a first type of a magnetic nanobead of a first type and/or of a non-magnetic nanobead,
• a binding site of a second type of a magnetic nanobead of a second type forms a binding of a second type with a complementary binding site of a second type of a non-magnetic nanobead,
• a binding site of a third type of a marker particle forms a binding of a third type with a complementary binding site of a third type of a non-magnetic nanobead,
iii. creating a second fluid
o by mixing the test fluid with the first nanobead fluid and mixing at least the first magnetic nanobead of the resulting fluid with the third nanobead fluid, whereupon the resulting fluid is referred to as the second fluid,
iv. removing the first magnetic nanobead from the second fluid by means of a first magnetic force,
o wherein, in the first case, the test substance particle bound to the first magnetic nanobead and the non-magnetic nanobead bound to the test substance particle are also removed from the second fluid
o or, in the second case, the non-magnetic nanobead remains in the second fluid,
v. creating a third fluid
o by mixing the second nanobead fluid and the marker fluid into the second fluid, whereupon the mixture is referred to as the third fluid,
vi. removing the second magnetic nanobead from the third fluid by means of a second magnetic force,
o wherein, in the first case, due to the absence of the non-magnetic nanobead in the third fluid, the marker particle is not indirectly bound to the second magnetic nanobead, and therefore it remains in the third fluid after the second magnetic nanobead is removed, or
o wherein, in the second case, the non-magnetic nanobead is bound to the second magnetic nanobead and the marker particle is bound to the non-magnetic nanobead, and therefore the marker particle is removed from the third fluid together with the second magnetic nanobead and the non-magnetic marker particle.

2. The method according to claim 1, wherein the third nanobead fluid is mixed with the test fluid and with the first nanobead fluid in order to create the second fluid.

3. The method according to claim 1, wherein, in order to create the second fluid,
• firstly, the first nanobead fluid is mixed with the test fluid,
• secondly, the first magnetic nanobead is removed from the test fluid by means of a third magnetic force, and
• thirdly, the first magnetic nanobead removed from the test fluid is introduced into the third nanobead fluid by means of a fourth magnetic force.

4. The method according to any one of claims 1 to 3, wherein
• the test substance particle has two or more binding sites of a first type,
• the first magnetic nanobead has a single or more complementary binding sites of a first type,
• the second magnetic nanobead has a single or more binding sites of a second type,
• the non-magnetic nanobead has a single or more complementary binding sites of a first type, a single or more complementary binding sites of a second type, a single or more complementary binding sites of a third type, and
• the marker particle has a single or more binding sites of a third type.

5. The method according to any one of claims 1 to 4, wherein
• the test fluid contains a single or multiple test substance particles and/or
• the first nanobead fluid contains a single or multiple first magnetic nanobeads and/or
• the second nanobead fluid contains a single or multiple second magnetic nanobeads and/or
• the third nanobead fluid contains a single or multiple non-magnetic nanobeads and/or
• the first marker fluid contains a single or multiple marker particles.

6. The method according to any one of claims 1 to 5, wherein the binding of a first type is an antibody-antigen binding or an oligonucleotide binding or a lipid binding or a glucose binding.

7. The method according to any one of claims 1 to 6, wherein the binding of a second type is an antibody-antigen binding or an oligonucleotide binding or a lipid binding or a glucose binding.

8. The method according to any one of claims 1 to 7, wherein the binding of a third type is an antibody-antigen binding or an oligonucleotide binding or a lipid binding or a glucose binding.

9. The method according to any one of claims 1 to 9, wherein the marker particle is a colored particle, a luminous particle, a fluorescent particle, a particle that influences the electrical conductivity of a liquid, or a radioactive particle.

10. The method according to any one of claims 1 to 10, wherein
• an additional test substance particle with an alternative binding site of a first type is located in the test fluid,
• an additional first nanobead fluid is provided which contains an alternative first nanobead with an alternative complementary binding site of a first type, and
• an additional third nanobead fluid is provided which contains an alternative non-magnetic nanobead with an alternative complementary binding site of a first type and an alternative complementary binding site of a third type, and
• an additional marker fluid is provided which contains an alternative marker particle with an alternative binding site of a third type,
• wherein the additional first nanobead fluid, the additional third nanobead fluid, and the additional marker fluid are used in the method in the same steps and in the same way as the first nanobead fluid, the third nanobead fluid, and the marker fluid, respectively.

11. The method according to claim 10, wherein
• the test fluid contains a single or multiple additional test substance particles and/or
• the first additional nanobead fluid contains a single or multiple alternative first nanobeads and/or
• the third additional nanobead fluid contains a single or multiple alternative third nanobeads and/or
• the additional marker fluid contains a single or multiple alternative marker particles.

12. The method according to any one of claims 1 to 11, wherein
• the first magnetic force is applied by means of a first magnet and/or
• the second magnetic force is applied by means of a second magnet and/or
• wherein
o the relevant magnet is immersed in the relevant fluid,
o as a result of magnetic forces, the relevant magnetic nanobead moves toward the relevant magnet and is then held on the relevant magnet,
o such that the magnet and nanobead are removed together from the fluid in order to remove the nanobead.

13. The method according to claim 3, wherein, when creating the second fluid,
• the third magnetic force is applied by means of a third magnet
o which is immersed in the test fluid,
o as a result of magnetic forces, the first magnetic nanobead moves toward the third magnet and is then held on the third magnet,
o such that the magnet and first magnetic nanobead are removed together from the fluid in order to remove the first magnetic nanobead, and
• the fourth magnetic force is generated by reversing the polarity of the third magnet, such that the first magnetic nanobead held on the magnet is repelled when the second fluid is created, wherein, in order to introduce the nanobead into the third nanobead fluid, the first magnet is first immersed in the third nanobead fluid before the polarity of the magnet is reversed.

14. The method according to claim 3, wherein, when creating the second fluid,
• the third magnetic force is applied by means of a third magnet
o which is immersed in the test fluid,
o as a result of magnetic forces, the first magnetic nanobead moves toward the third magnet and is then held on the third magnet,
o such that the magnet and first magnetic nanobead are removed together from the fluid in order to remove the first magnetic nanobead, and
• the fourth magnetic force is generated by means of a magnetic field impressed on the third nanobead fluid from outside, wherein the fourth magnetic force is only impressed after the third magnet, with the first magnetic nanobead held on the third magnet, has been immersed in the third nanobead fluid.

15. The method according to any one of claims 12 to 14, wherein the first and/or second and/or third magnet is surrounded by a protective layer, such that the relevant magnetic nanobead is held on the protective layer as a result of the magnetic forces.

16. The method according to any one of claims 1 to 15, wherein the number of test substance particles originally present in the test fluid is determined in that
• a known number of non-magnetic nanobeads, in each case with a known number of complementary binding sites of a first and third type, is used,
o wherein the total number of complementary binding sites of a first type present on the non-magnetic nanobeads is greater than the number of test substance particles
o and wherein the total number of complementary binding sites of a third type present on the non-magnetic nanobeads is less than the number of marker particles, and
• the difference between the marker particles remaining in the third fluid at the end and the marker particles present at the start is determined,
• the number of test substances originally present is calculated using the difference of the marker particles with knowledge of the number of non-magnetic nanobeads.

17. The method according to any one of claims 1 to 16, wherein the container in which the second fluid is created tapers conically downward.

18. The method according to any one of claims 1 to 17, which is used to detect the test substance in the test fluid if the number of test substance particles is within the femto- and/or atto- and/or zeptomolar range.

19. The method according to any one of claims 1 to 18, which is used for single-molecule counting.

## Revendications

1. Méthode de détection directe de la présence d'une substance d'essai dans un fluide d'essai comprenant les étapes suivantes :
i. Fourniture d'un premier fluide, le fluide d'essai, dans lequel est présente dans un premier cas une particule de substance d'essai, et dans un second cas la particule de substance d'essai n'est pas présente,
o sachant que la particule de substance d'essai comporte des sites de liaison de premier type,
ii. Fourniture
o d'un premier fluide de nanobilles qui contient une première nanobille magnétique comportant un site de liaison complémentaire de premier type,
o d'un deuxième fluide de nanobilles qui contient une deuxième nanobille magnétique comportant un site de liaison de deuxième type,
o d'un troisième fluide de nanobilles qui contient une nanobille non-magnétique comportant un site de liaison complémentaire de premier type, un site de liaison complémentaire de deuxième type et un site de liaison complémentaire de troisième type,
o d'un fluide marqueur, qui contient une particule marqueuse comportant un site de liaison de troisième type,
o sachant que, en présence des partenaires de liaison correspondants dans le même fluide,
▪ un site de liaison de premier type d'une particule de substance d'essai avec un site de liaison complémentaire de premier type d'une nanobille magnétique de premier type et/ou d'une nanobille non magnétique forme une liaison de premier type.,
▪ un site de liaison de deuxième type d'une nanobille magnétique de deuxième type avec un site de liaison complémentaire de deuxième type d'une nanobille non magnétique forme une liaison de deuxième type,
▪ un site de liaison de troisième type d'une particule marqueuse avec un site de liaison complémentaire de troisième type d'une nanobille non magnétique forme une liaison de troisième type,
iii. Préparation d'un deuxième fluide,
o en mélangeant le fluide d'essai avec le premier fluide de nanobilles et en mélangeant au moins la première nanobille magnétique du fluide produit avec le troisième fluide de nanobilles, le fluide en résultant étant désigné comme le deuxième fluide,
iv. Retrait de la première nanobille magnétique du deuxième fluide au moyen d'une première force magnétique,
o sachant que, dans le premier cas, la particule de substance d'essai reliée à la première nanobille magnétique et la nanobille non-magnétique reliée à la particule de substance d'essai sont également extraites du deuxième fluide
o ou, dans le deuxième cas, la nanobille non-magnétique demeure dans le deuxième fluide,
v. Préparation d'un troisième fluide,
o en incorporant le deuxième fluide de nanobilles et le fluide marqueur dans le deuxième fluide, le mélange étant désigné comme le troisième fluide,
vi. Retrait de la deuxième nanobille magnétique du troisième fluide au moyen d'une deuxième force magnétique,
o sachant que, dans le premier cas, en raison de l'absence de la nanobille non-magnétique dans le troisième fluide, la particule marqueuse n'est pas liée indirectement à la deuxième nanobille magnétique, de sorte qu'elle demeure dans le troisième fluide après le retrait de la deuxième nanobille magnétique, ou
o sachant que, dans le deuxième cas, la nanobille non-magnétique est liée à la deuxième nanobille magnétique et la particule marqueuse est liée à la nanobille non-magnétique, de sorte que la particule marqueuse est extraite du troisième fluide conjointement à la deuxième nanobille magnétique et à la particule marqueuse non-magnétique.

2. Méthode selon la revendication 1, dans laquelle, pour préparer le deuxième fluide, le troisième fluide de nanobilles est mélangé avec le fluide d'essai et avec le premier fluide de nanobilles.

3. Méthode selon la revendication 1, dans laquelle, pour préparer le deuxième fluide,
• premièrement, le premier fluide de nanobilles est mélangé avec le fluide d'essai,
• deuxièmement, la première nanobille magnétique est extraite du fluide d'essai par une troisième force magnétique et
• troisièmement, la première nanobille magnétique extraite du fluide d'essai est déposée dans le troisième fluide de nanobilles par une quatrième force magnétique.

4. Méthode selon l'une des revendications 1 à 3, dans laquelle
• la particule de substance d'essai comporte deux ou plusieurs sites de liaison de premier type,
• la première nanobille magnétique comporte un seul ou plusieurs sites de liaison complémentaires de premier type,
• la deuxième nanobille magnétique comporte un seul ou plusieurs sites de liaison de deuxième type,
• la nanobille non-magnétique comporte un seul ou plusieurs sites de liaison complémentaires de premier type, un seul ou plusieurs sites de liaison complémentaires de deuxième type, un seul ou plusieurs sites de liaison complémentaires de troisième type, de même que
• la particule marqueuse comporte un seul ou plusieurs sites de liaison de troisième type.

5. Méthode selon l'une des revendications 1 à 4, dans laquelle
• le fluide d'essai contient une seule ou plusieurs particules de substance d'essai et/ou
• le premier fluide de nanobilles contient une seule ou plusieurs premières nanobilles magnétiques et/ou
• le deuxième fluide de nanobilles contient une seule ou plusieurs deuxièmes nanobilles magnétiques et/ou
• le troisième fluide de nanobilles contient une seule ou plusieurs nanobilles non-magnétiques et/ou
• le premier fluide marqueur contient une seule ou plusieurs particules marqueuses.

6. Méthode selon l'une des revendications 1 à 5, dans laquelle la liaison de premier type est une liaison anticorps-antigène ou une liaison oligonucléotidique ou une liaison lipidique ou une liaison au glucose.

7. Méthode selon l'une des revendications 1 à 6, dans laquelle la liaison de deuxième type est une liaison anticorps-antigène ou une liaison oligonucléotidique ou une liaison lipidique ou une liaison au glucose.

8. Méthode selon l'une des revendications 1 à 7, dans laquelle la liaison de troisième type est une liaison anticorps-antigène ou une liaison oligonucléotidique ou une liaison lipidique ou une liaison au glucose.

9. Méthode selon l'une des revendications 1 à 9, dans laquelle la particule marqueuse est une particule colorée, une particule lumineuse, une particule fluorescente, une particule influençant la conductivité électrique d'un liquide ou une particule radioactive.

10. Méthode selon l'une des revendications 1 à 10, dans laquelle
• une particule de substance d'essai supplémentaire comportant un site de liaison alternatif de premier type se trouve dans le fluide d'essai,
• un premier fluide de nanobilles supplémentaire est fourni, lequel contient une première nanobille alternative comportant un site de liaison complémentaire alternatif de premier type, et
• un troisième fluide de nanobilles supplémentaire est fourni, lequel contient une nanobille non-magnétique alternative comportant un site de liaison complémentaire alternatif de premier type et un site de liaison complémentaire alternatif de troisième type, et
• un fluide marqueur supplémentaire est fourni, lequel contient une particule marqueuse alternative comportant un site de liaison alternatif de troisième type,
• sachant que le premier fluide de nanobilles supplémentaire, le troisième fluide de nanobilles supplémentaire et le fluide marqueur supplémentaire sont utilisés dans la méthode avec les mêmes étapes et de la même manière que le premier fluide de nanobilles, le troisième fluide de nanobilles, respectivement le fluide marqueur.

11. Méthode selon la revendication 10, dans laquelle
• le fluide d'essai contient une seule ou plusieurs particules de substance d'essai supplémentaires, et/ou
• le premier fluide de nanobilles supplémentaire contient une seule ou plusieurs premières nanobilles alternatives et/ou
• le troisième fluide de nanobilles supplémentaire contient une seule ou plusieurs troisièmes nanobilles alternatives et/ou
• le fluide marqueur supplémentaire contient une seule ou plusieurs particules marqueuses alternatives.

12. Méthode selon l'une des revendications 1 à 11, dans laquelle
• la première force magnétique est appliquée par un premier aimant et/ou
• la deuxième force magnétique est appliquée par un deuxième aimant et/ou
• sachant que
o l'aimant respectif est immergé dans le fluide respectif,
o sous l'effet des forces magnétiques, la nanobille magnétique respective se déplace vers l'aimant respectif et est ensuite maintenue contre celui-ci,
o de sorte que l'aimant et la nanobille sont extraits conjointement du fluide en vue du retrait de la nanobille.

13. Méthode selon la revendication 3, dans laquelle, lors de la préparation du deuxième fluide
• la troisième force magnétique est appliquée par un troisième aimant,
o qui est immergé dans le fluide d'essai,
o sous l'effet des forces magnétiques, la première nanobille magnétique se déplace vers le troisième aimant et est ensuite maintenue contre celui-ci,
o de sorte que l'aimant et la première nanobille magnétique sont extraits conjointement du fluide en vue du retrait de la première nanobille magnétique, et
• la quatrième force magnétique est produite par inversion de polarité du troisième aimant, de sorte que, lors de la préparation du deuxième fluide, la première nanobille magnétique maintenue contre l'aimant est repoussée, sachant que, pour introduire la nanobille dans le troisième fluide de nanobilles, le premier aimant est d'abord immergé dans le troisième fluide de nanobilles avant que la polarité de l'aimant soit inversée.

14. Méthode selon la revendication 3, dans laquelle, lors de la préparation du deuxième fluide
• la troisième force magnétique est appliquée par un troisième aimant,
o qui est immergé dans le fluide d'essai,
o sous l'effet des forces magnétiques, la première nanobille magnétique se déplace vers le troisième aimant et est ensuite maintenue contre celui-ci,
o de sorte que l'aimant et la première nanobille magnétique sont extraits conjointement du fluide en vue du retrait de la première nanobille magnétique, et
• la quatrième force magnétique est produite par un champ magnétique appliqué de l'extérieur sur le troisième fluide de nanobilles, sachant que la quatrième force magnétique est appliquée seulement après l'immersion, dans le troisième fluide de nanobilles, du troisième aimant avec la première nanobille magnétique maintenue contre ce troisième aimant.

15. Méthode selon l'une des revendications 12 à 14, dans laquelle le premier et/ou le deuxième et/ou le troisième aimant est entouré d'une couche de protection, de sorte que la nanobille magnétique respective est maintenue sous l'effet des forces magnétiques contre la couche de protection.

16. Méthode selon l'une des revendications 1 à 15, dans laquelle le nombre de particules de substance d'essai existant à l'origine dans le fluide d'essai est défini en
• utilisant un nombre connu de nanobilles non-magnétiques comportant un nombre connu respectif de sites de liaison complémentaires de premier et troisième types,
o sachant que le nombre de sites de liaison complémentaires de premier type présents au total sur les nanobilles non-magnétiques est supérieur au nombre de particules de substance d'essai,
o et sachant que le nombre de sites de liaison complémentaires de troisième type présents au total sur les nanobilles non-magnétiques est inférieur au nombre de particules marqueuses, et
• déterminant la différence entre les particules marqueuses résiduelles à la fin dans le troisième fluide et les particules marqueuses présentes au début,
• calculant le nombre des substances d'essai présentes à l'origine au moyen de la différence des particules marqueuses compte tenu du nombre de nanobilles non-magnétiques.

17. Méthode selon l'une des revendications 1 à 16, dans laquelle le récipient, dans lequel est préparé le deuxième fluide, présente une forme conique vers le bas.

18. Méthode selon l'une des revendications 1 à 17, laquelle est mise en œuvre pour la détection de la substance d'essai dans le fluide d'essai, lorsque le nombre de particules de substance d'essai se situe dans le domaine femto-, et/ou atto- et/ou zeptomolaire.

19. Méthode selon l'une des revendications 1 à 18, laquelle est mise en œuvre pour le comptage de molécules uniques (Single-Molecule-Counting).
